(19) 

Europäisches
Patentamt
European
Patent Office
Office européen
des brevets

(11) **EP 4 053 157 A1**

(12) **EUROPEAN PATENT APPLICATION**
published in accordance with Art. 153(4) EPC

(43) Date of publication:
07.09.2022 Bulletin 2022/36

(21) Application number: 20883248.5

(22) Date of filing: 02.11.2020

(51) International Patent Classification (IPC):
*C07K 14/47* (2006.01)    *C07K 16/28* (2006.01)
*A61K 39/00* (2006.01)    *A61K 39/385* (2006.01)
*A61K 9/51* (2006.01)    *A61K 9/19* (2006.01)
*A61K 9/00* (2006.01)    *A61P 35/00* (2006.01)
*B82Y 5/00* (2011.01)

(52) Cooperative Patent Classification (CPC):
A61K 9/00; A61K 9/19; A61K 9/51; A61K 39/00;
A61K 39/385; A61P 35/00; C07K 14/47;
C07K 16/28; B82Y 5/00

(86) International application number:
PCT/KR2020/015164

(87) International publication number:
WO 2021/086158 (06.05.2021 Gazette 2021/18)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 01.11.2019 KR 20190138580

(71) Applicant: Cellemedy Co., Ltd
**Gyeonggi-do 13605 (KR)**

(72) Inventors:
• LEE, Jee Won
  **Seoul 06707 (KR)**
• LEE, Bo Ram
  **Seoul 02476 (KR)**
• YOON, Chul Joo
  **Seoul 06599 (KR)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **DISEASE ANTIGEN-FUSED PROTEIN, AND USE THEREOF**

(57)    The present invention relates to a protein fused with a disease antigen and a use of same. The protein of the present invention is formed via self-assembly of ferritin monomers fused with a disease antigen epitope. The protein of the present invention exhibits excellent binding affinity to a human transferrin receptor, and thus can provide various kinds of disease antigen epitopes with different lengths to antigen-presenting cells so as to induce an immune response to the corresponding antigen.

EP 4 053 157 A1

[FIG. 12]

<110>    Cellemedy Co. Ltd

<120>    ANTIGEN FUSED PROTEIN AND USE THEREOF

<130>    20P11003

<150>    KR 10-2019-0138580
<151>    2019-11-01

<150>    KR 10-2020-0144570
<151>    2020-11-02

<160>    67

<170>    KoPatentIn 3.0

<210>    1
<211>    183
<212>    PRT
<213>    Homo sapiens

<400>    1
Met Thr Thr Ala Ser Thr Ser Gln Val Arg Gln Asn Tyr His Gln Asp
1               5                   10                  15

Ser Glu Ala Ala Ile Asn Arg Gln Ile Asn Leu Glu Leu Tyr Ala Ser
            20                  25                  30

Tyr Val Tyr Leu Ser Met Ser Tyr Tyr Phe Asp Arg Asp Asp Val Ala
            35                  40                  45

Leu Lys Asn Phe Ala Lys Tyr Phe Leu His Gln Ser His Glu Glu Arg
        50                  55                  60

Glu His Ala Glu Lys Leu Met Lys Leu Gln Asn Gln Arg Gly Gly Arg
65                  70                  75                  80

Ile Phe Leu Gln Asp Ile Lys Lys Pro Asp Cys Asp Asp Trp Glu Ser
                85                  90                  95

Gly Leu Asn Ala Met Glu Cys Ala Leu His Leu Glu Lys Asn Val Asn
            100                 105                 110

Gln Ser Leu Leu Glu Leu His Lys Leu Ala Thr Asp Lys Asn Asp Pro
            115                 120                 125

His Leu Cys Asp Phe Ile Glu Thr His Tyr Leu Asn Glu Gln Val Lys
        130                 135                 140

Ala Ile Lys Glu Leu Gly Asp His Val Thr Asn Leu Arg Lys Met Gly
145                 150                 155                 160

Ala Pro Glu Ser Gly Leu Ala Glu Tyr Leu Phe Asp Lys His Thr Leu
                165                 170                 175

Gly Asp Ser Asp Asn Glu Ser
            180

<210>    2
<211>    9
<212>    PRT
<213>    Homo sapiens

<400>    2
Lys Val Pro Arg Asn Gln Asp Trp Leu
  1               5


<210>    3
<211>    8
<212>    PRT
<213>    Homo sapiens

<400>    3
Ser Ile Ile Asn Phe Glu Lys Leu
  1               5


<210>    4
<211>    9
<212>    PRT
<213>    Homo sapiens

<400>    4
Ser Pro Ser Tyr Val Tyr His Gln Phe
  1               5


<210>    5
<211>    149
<212>    PRT
<213>    Homo sapiens

<400>    5
Gly Trp Leu Leu Glu Val Pro Asn Gly Pro Trp Arg Ser Leu Thr Phe
  1               5                   10                  15

Tyr Pro Ala Trp Leu Thr Val Ser Glu Gly Ala Asn Ala Thr Phe Thr
              20                  25                  30

Cys Ser Leu Ser Asn Trp Ser Glu Asp Leu Met Leu Asn Trp Asn Arg
              35                  40                  45

Leu Ser Pro Ser Asn Gln Thr Glu Lys Gln Ala Ala Phe Cys Asn Gly
          50                  55                  60

Leu Ser Gln Pro Val Gln Asp Ala Arg Phe Gln Ile Ile Gln Leu Pro
 65                  70                  75                  80

Asn Arg His Asp Phe His Met Asn Ile Leu Asp Thr Arg Arg Asn Asp
                  85                  90                  95

Ser Gly Ile Tyr Leu Cys Gly Ala Ile Ser Leu His Pro Lys Ala Lys
              100                 105                 110

Ile Glu Glu Ser Pro Gly Ala Glu Leu Val Val Thr Glu Arg Ile Leu
              115                 120                 125

Glu Thr Ser Thr Arg Tyr Pro Ser Pro Ser Pro Lys Pro Glu Gly Arg
          130                 135                 140

Phe Gln Gly Met Val
145

```
<210>      6
<211>      22
<212>      PRT
<213>      Homo sapiens

<400>      6
Ser Gly Gln Tyr Ala Ser Tyr His Cys Trp Cys Trp Arg Asp Pro Gly
 1               5                  10                  15

Arg Ser Gly Gly Ser Lys
            20


<210>      7
<211>      12
<212>      PRT
<213>      Homo sapiens

<400>      7
Ser Gly His Gly Tyr Ser Tyr Gly Ala Phe Asp Tyr
 1               5                  10


<210>      8
<211>      52
<212>      PRT
<213>      Unknown

<220>
<223>      Mouse


<400>      8
Met Leu Asn Trp Asn Arg Leu Ser Pro Ser Asn Gln Thr Glu Lys Gln
 1               5                  10                  15

Ala Ala Phe Cys Asn Gly Gly Gly Ser Gly Gly Gly Thr Gly Gly Gly
            20                  25                  30

Ser Gly Ile Tyr Leu Cys Gly Ala Ile Ser Leu His Pro Lys Ala Lys
            35                  40                  45

Ile Glu Asp Ser
         50


<210>      9
<211>      225
<212>      PRT
<213>      Artificial Sequence

<220>
<223>      RFP antigen


<400>      9
Met Asp Asn Thr Glu Asp Val Ile Lys Glu Phe Met Gln Phe Lys Val
 1               5                  10                  15

Arg Met Glu Gly Ser Val Asn Gly His Tyr Phe Glu Ile Glu Gly Glu
            20                  25                  30

Gly Glu Gly Lys Pro Tyr Glu Gly Thr Gln Thr Ala Lys Leu Gln Val
```

|  | 35 |  |  |  |  | 40 |  |  |  |  | 45 |  |  |
|---|---|---|---|---|---|---|---|---|---|---|---|---|---|

Thr Lys Gly Gly Pro Leu Pro Phe Ala Trp Asp Ile Leu Ser Pro Gln
    50                  55              60

Phe Gln Tyr Gly Ser Lys Ala Tyr Val Lys His Pro Ala Asp Ile Pro
    65              70              75                  80

Asp Tyr Met Lys Leu Ser Phe Pro Glu Gly Phe Thr Trp Glu Arg Ser
            85                  90                  95

Met Asn Phe Glu Asp Gly Gly Val Val Glu Val Gln Gln Asp Ser Ser
            100                 105                 110

Leu Gln Asp Gly Thr Phe Ile Tyr Lys Val Lys Phe Lys Gly Val Asn
        115                 120                 125

Phe Pro Ala Asp Gly Pro Val Met Gln Lys Lys Thr Ala Gly Trp Glu
    130                 135                 140

Pro Ser Thr Glu Lys Leu Tyr Pro Gln Asp Gly Val Leu Lys Gly Glu
145                 150                 155                 160

Ile Ser His Ala Leu Lys Leu Lys Asp Gly Gly His Tyr Thr Cys Asp
            165                 170                 175

Phe Lys Thr Val Tyr Lys Ala Lys Lys Pro Val Gln Leu Pro Gly Asn
            180                 185                 190

His Tyr Val Asp Ser Lys Leu Asp Ile Thr Asn His Asn Glu Asp Tyr
        195                 200                 205

Thr Val Val Glu Gln Tyr Glu His Ala Glu Ala Arg His Ser Gly Ser
    210                 215                 220

Gln
225


```
<210>    10
<211>    72
<212>    DNA
<213>    Artificial Sequence

<220>
<223>    N gp100 F


<400>    10
catatgcacc atcaccatca ccataaggtt ccacgtaatc aagactggct ggaattcacg      60

accgcgtcca cc                                                          72


<210>    11
<211>    27
<212>    DNA
<213>    Artificial Sequence

<220>
<223>    N gp100 R
```

<400>    11
aagcttctag ctttcattat cactgtc                                                    27


<210>    12
<211>    38
<212>    DNA
<213>    Artificial Sequence

<220>
<223>    AB gp100 F


<400>    12
tcaagactgg ctggaattcg tggctttgaa gaactttg                                        38


<210>    13
<211>    39
<212>    DNA
<213>    Artificial Sequence

<220>
<223>    AB gp100 R


<400>    13
ttacgtggaa ccttggatcc atcatcgcgg tcaaagtag                                       39


<210>    14
<211>    37
<212>    DNA
<213>    Artificial Sequence

<220>
<223>    BC gp100 F


<400>    14
tcaagactgg ctggaattct gggagagcgg gctgaat                                         37


<210>    15
<211>    47
<212>    DNA
<213>    Artificial Sequence

<220>
<223>    BC gp100 R


<400>    15
ttacgtggaa ccttggatcc gtcatcacag tctggtttct tgatatc                             47


<210>    16
<211>    37
<212>    DNA
<213>    Artificial Sequence

<220>
<223>    CD gp100 F

```
<400>    16
tcaagactgg ctggaattcc cccatttgtg tgacttc                                    37


<210>    17
<211>    38
<212>    DNA
<213>    Artificial Sequence


<220>
<223>    CD gp100 R


<400>    17
ttacgtggaa ccttggatcc gtcatttttg tcagtggc                                   38


<210>    18
<211>    36
<212>    DNA
<213>    Artificial Sequence


<220>
<223>    DE gp100 F


<400>    18
catatgcacc atcaccatca ccatctcgag acgacc                                     36


<210>    19
<211>    48
<212>    DNA
<213>    Artificial Sequence


<220>
<223>    DE gp100 R1


<400>    19
aagcttctag ctttcattat cactgtctcc cagggtgtgc ttgtcaaa                        48


<210>    20
<211>    48
<212>    DNA
<213>    Artificial Sequence


<220>
<223>    DE gp100 R2


<400>    20
gtgcttgtca aagagatatt ccgccaatcc agagaattcc agccagtc                        48


<210>    21
<211>    48
<212>    DNA
<213>    Artificial Sequence
```

<220>
<223>    DE gp100 R3

<400>    21
ttccagccag tcttgattac gtggaacctt ggatccttcg ggcgctcc                    48

<210>    22
<211>    39
<212>    DNA
<213>    Artificial Sequence

<220>
<223>    C gp100 F

<400>    22
catatgacga ccgcgtccac ctcgcaggtg cgccagaac                              39

<210>    23
<211>    66
<212>    DNA
<213>    Artificial Sequence

<220>
<223>    C gp100 R

<400>    23
aagcttctaa tggtgatggt gatggtgcag ccagtcttga ttacgtggaa ccttgctttc      60

attatc                                                                  66

<210>    24
<211>    36
<212>    DNA
<213>    Artificial Sequence

<220>
<223>    NA gp100 F1

<400>    24
catatgcacc atcaccatca ccatacgacc gcgtcc                                 36

<210>    25
<211>    33
<212>    DNA
<213>    Artificial Sequence

<220>
<223>    NA gp100 F2

<400>    25
ccacgtaatc aagactggct gcaggtgcgc cag                                    33

<210>    26

32

<211> 36
<212> DNA
<213> Artificial Sequence

<220>
<223> NA gp100 F3

<400> 26
acgaccgcgt ccacctcgaa ggttccacgt aatcaa                                    36

<210> 27
<211> 36
<212> DNA
<213> Artificial Sequence

<220>
<223> NA gp100 R

<400> 27
aagcttctag ctttcattat cactgtctcc cagggt                                    36

<210> 28
<211> 42
<212> DNA
<213> Artificial Sequence

<220>
<223> intD gp100 F

<400> 28
catatgcacc atcaccatca ccatgaattc acgaccgcgt cc                             42

<210> 29
<211> 48
<212> DNA
<213> Artificial Sequence

<220>
<223> intD gp100 R1

<400> 29
tcccatcttc agccagtctt gattacgtgg aaccttgcgc aagttggt                       48

<210> 30
<211> 45
<212> DNA
<213> Artificial Sequence

<220>
<223> intD gp100 R2

<400> 30
aaagagatat tccgccaatc cagattcggg cgctcccatc ttcag                          45

```
<210>    31
<211>    42
<212>    DNA
<213>    Artificial Sequence

<220>
<223>    intE gp100 F


<400>    31
catatgcacc atcaccatca ccatgaattc acgaccgcgt cc                          42


<210>    32
<211>    39
<212>    DNA
<213>    Artificial Sequence

<220>
<223>    intE gp100 R1


<400>    32
cagccagtct tgattacgtg gaaccttgtg cttgtcaaa                              39


<210>    33
<211>    48
<212>    DNA
<213>    Artificial Sequence

<220>
<223>    intE gp100 R2


<400>    33
aagcttctag ctttcattat cactgtctcc cagggtcagc cagtcttg                    48


<210>    34
<211>    42
<212>    DNA
<213>    Artificial Sequence

<220>
<223>    EC gp100 F


<400>    34
catatgcacc atcaccatca ccatgaattc acgaccgcgt cc                          42


<210>    35
<211>    60
<212>    DNA
<213>    Artificial Sequence

<220>
<223>    EC gp100 R


<400>    35
```

```
aagcttctag ctttcattat ccagccagtc ttgattacgt ggaaccttac tgtctcccag        60

                                                                          60
```

<210>    36
<211>    8
<212>    PRT
<213>    Artificial Sequence

<220>
<223>    linker1

<400>    36
Gly Gly Gly Ser Gly Gly Gly Thr
 1               5

<210>    37
<211>    10
<212>    PRT
<213>    Artificial Sequence

<220>
<223>    linker2

<400>    37
Gly Gly Gly Gly Ser Gly Gly Gly Gly Thr
 1               5                   10

<210>    38
<211>    12
<212>    PRT
<213>    Artificial Sequence

<220>
<223>    linker3

<400>    38
Gly Gly Gly Ser Gly Gly Gly Thr Gly Gly Gly Ser
 1               5                   10

<210>    39
<211>    29
<212>    DNA
<213>    Artificial Sequence

<220>
<223>    F primer

<400>    39
```
tgcagatatc aagaaaccag actgtgatg                                          29
```

<210>    40
<211>    23
<212>    DNA

<213>    Artificial Sequence

<220>
<223>    R primer

<400>    40
agcgcgattc ggccacctcg ttg                                                    23

<210>    41
<211>    52
<212>    PRT
<213>    Homo sapiens

<400>    41
Val Leu Asn Trp Tyr Arg Met Ser Pro Ser Asn Gln Thr Asp Lys Leu
 1               5                   10                  15

Ala Ala Phe Pro Glu Gly Gly Gly Ser Gly Gly Gly Thr Gly Gly Gly
            20                  25                  30

Ser Gly Thr Tyr Leu Cys Gly Ala Ile Ser Leu Ala Pro Lys Ala Gln
            35                  40                  45

Ile Lys Glu Ser
        50

<210>    42
<211>    12
<212>    PRT
<213>    Homo sapiens

<400>    42
Gly Gly Ser Tyr Gly Ser Leu Tyr Ala Phe Asp Ile
 1               5                   10

<210>    43
<211>    19
<212>    PRT
<213>    Homo sapiens

<400>    43
Met Pro Ser Phe Ile Thr Leu Ala Ser Leu Ser Thr Trp Glu Gly Tyr
 1               5                   10                  15

Phe Asp Phe

<210>    44
<211>    42
<212>    DNA
<213>    Artificial Sequence

<220>
<223>    BC alpha PDL1 F

<400>    44
ctgtatgcgt ttgatattga attctgggag agcgggctga at                               42

36

<210> 45
<211> 51
<212> DNA
<213> Artificial Sequence

<220>
<223> BC alpha PDL1 R

<400> 45
agaaccataa gaaccaccgg atccgtcatc acagtctggt ttcttgatat c            51

<210> 46
<211> 36
<212> DNA
<213> Artificial Sequence

<220>
<223> C alpha TIGIT F

<400> 46
catatgcacc atcaccatca ccatacgacc gcgtcc            36

<210> 47
<211> 78
<212> DNA
<213> Artificial Sequence

<220>
<223> C alpha TIGIT R

<400> 47
aagcttctaa aaatcaaaat agccttccca ggtgctcagg ctcgccaggg taataaagct            60

cggcatgctt tcattatc            78

<210> 48
<211> 12
<212> PRT
<213> Homo sapiens

<400> 48
Gly Gly Ser Tyr Gly Ser Leu Tyr Ala Phe Asp Ile
1               5                   10

<210> 49
<211> 13
<212> PRT
<213> Homo sapiens

<400> 49
Asp Leu Gly Ala Gly Pro Tyr Tyr Tyr Gly Lys Asp Val
1               5                   10

<210> 50
<211> 6
<212> PRT
<213> Homo sapiens

<400> 50
Thr Ala Gln Phe Ala Tyr
1               5

<210> 51
<211> 12
<212> PRT
<213> Homo sapiens

<400> 51
Gly Tyr Ser Asp Tyr Glu Tyr Asn Trp Phe Asp Pro
1               5                   10

<210> 52
<211> 9
<212> PRT
<213> Homo sapiens

<400> 52
Val Gly Gly Ala Phe Pro Met Asp Tyr
1               5

<210> 53
<211> 19
<212> PRT
<213> Homo sapiens

<400> 53
Met Pro Ser Phe Ile Thr Leu Ala Ser Leu Ser Thr Trp Glu Gly Tyr
1               5                   10                  15

Phe Asp Phe

<210> 54
<211> 36
<212> DNA
<213> Artificial Sequence

<220>
<223> alpha PDL1 F

<400> 54
catatgcacc atcaccatca ccatacgacc gcgtcc                                    36

<210> 55
<211> 57
<212> DNA
<213> Artificial Sequence

<220>
<223> alpha PDL1 R

<400>    55
aagcttctaa atatcaaacg catacagaga accataagaa ccaccgcttt cattatc                57


<210>    56
<211>    36
<212>    DNA
<213>    Artificial Sequence

<220>
<223>    alpha PD1 F


<400>    56
catatgcacc atcaccatca ccatacgacc gcgtcc                                       36


<210>    57
<211>    60
<212>    DNA
<213>    Artificial Sequence

<220>
<223>    alpha PD1 R


<400>    57
aagcttctac acatctttgc catagtaata cgggcccgcg cccagatcgc tttcattatc            60

                                                                              60


<210>    58
<211>    36
<212>    DNA
<213>    Artificial Sequence

<220>
<223>    alpha CTLA4 F


<400>    58
catatgcacc atcaccatca ccatacgacc gcgtcc                                       36


<210>    59
<211>    45
<212>    DNA
<213>    Artificial Sequence

<220>
<223>    alpha CTLA4 R


<400>    59
aagcttctaa tacgcaaact gcgcggtctc gaggctttca ttatc                            45


<210>    60
<211>    36
<212>    DNA

<213>       Artificial Sequence

<220>
<223>       alpha LAG3 F


<400>       60
catatgcacc atcaccatca ccatacgacc gcgtcc                                    36


<210>       61
<211>       57
<212>       DNA
<213>       Artificial Sequence

<220>
<223>       alpha LAG3 R


<400>       61
aagcttctac ggatcaaacc agttatattc ataatcgcta tagccgcttt cattatc            57


<210>       62
<211>       36
<212>       DNA
<213>       Artificial Sequence

<220>
<223>       alpha TIM3 F


<400>       62
catatgcacc atcaccatca ccatacgacc gcgtcc                                    36


<210>       63
<211>       48
<212>       DNA
<213>       Artificial Sequence

<220>
<223>       alpha TIM3 R


<400>       63
aagcttctaa taatccatcg gaaatgcacc gccaacgctt tcattatc                       48


<210>       64
<211>       36
<212>       DNA
<213>       Artificial Sequence

<220>
<223>       alpha TIGIT F


<400>       64
catatgcacc atcaccatca ccatacgacc gcgtcc                                    36


<210>       65

```
<211>       78
<212>       DNA
<213>       Artificial Sequence

<220>
<223>       alpha TIGIT R


<400>       65
aagcttctaa aaatcaaaat agccttccca ggtgctcagg ctcgccaggg taataaagct       60

cggcatgctt tcattatc                                                     78


<210>       66
<211>       42
<212>       DNA
<213>       Artificial Sequence

<220>
<223>       BC alpha PDL1 F


<400>       66
ctgtatgcgt ttgatattga attctgggag agcgggctga at                          42


<210>       67
<211>       51
<212>       DNA
<213>       Artificial Sequence

<220>
<223>       BC alpha PDL1 R


<400>       67
agaaccataa gaaccaccgg atccgtcatc acagtctggt ttcttgatat c                51
```

**Description**

[Technical Field]

The present invention relates to a disease antigen-fused protein, and use thereof.

[Background Art]

In modern times, with the development of medical technology, non-curable diseases have almost disappeared, but cancer still requires very difficult and complex treatment unlike other disease treatment. Currently, methods used for cancer treatment include surgery, radiation therapy, and chemotherapy. If the cancer does not metastasize to other areas but develops locally, it can be treated through cancer removal surgery. However, since cancer metastasis occurs in 70% or more of cancer patients, adjuvant therapy should be combined.

As one of such adjuvant therapies, radiation therapy to kill cancer cells using high energy radiation is performed. The radiation therapy inhibits the proliferation of cancer cells by irradiating the cancer cells, such that new cancer cells cannot be generated while preventing further division thereof. However, this method has a problem of entailing side effects to affect not only cancer cells but also normal cells.

Chemotherapy is an adjuvant therapy in which a drug is used to kill cancer cells after surgery, and is performed for the purpose of killing invisible cancer cells. However, the chemotherapy has a problem that side effects such as vomiting, diarrhea, and hair loss are accompanied.

Immunotherapy methods have recently emerged to minimize these side effects. Immunotherapy is a method of treating cancer using the patient's immune response, and can even prevent cancer. Cancer immunotherapy is a treatment method that activates cancer-specific immune cells by administering an antigen causing tumor formation, as in the principle of a vaccine, and then allows the activated immune cells to specifically attack the cancer in the body. Further, even if not suffering from cancer, administering a cancer-specific antigen into the body may activate inactivated immune cells into cancer-specific memory immune cells, thereby specifically attacking cancer cells when cancer develops.

For cancer immunotherapy, it is important to transport cancer-specific antigens (tumor-associated antigen (TAA), tumor-specific antigen (TSA)) to lymph nodes where immune cells are concentrated. In particular, among tumor-specific antigens, neoantigens, which are found in various tumor types such as lung cancer and kidney cancer and mainly found in melanoma, can be newly generated by potential gene activity of individuals with cancer or mutations in the DNA part. These antigens are very important in producing a "customized cancer vaccine" based on patient's individual genetic information.

However, conventional attempts to transport only cancer-specific antigens to lymph nodes have not been very effective. The reason is that the tumor antigen itself was somewhat short in a length, and the tumor antigen presentation efficiency for amplifying and activating tumor antigen-specific immune cells was very low, which in turn considerably reduced the efficiency of inducing tumor antigen-specific immunity (Non-Patent Document 1). As carriers of the cancer-specific antigens as described above in the body, polymers are widely used. Further, if a cancer antigen is immobilized on the surface of a polymer for transporting a cancer-specific antigen in the body, the cancer-specific antigen should be exposed to the surface of particles through chemical binding. However, there is still a limitation in regard to uniform exposition of the cancer-specific antigen to the surface of particles at a high density.

Cancer immunotherapy uses the patient's immune system thus to involve low side effects, compared to conventional anti-cancer treatment methods, exerts therapeutic effects sustained for a long time by the formation of immune memory, and due to the principle of tumor antigen-specific recognition, less influences on general cells thus to have an advantage of very little side effects. Further, due to recent clinical success cases for cancer patients with recurrent or anticancer drug resistance, cancer immunotherapy has been receiving explosive attention enough to be selected by Science as Breakthrough of the year 2013.

[Detailed Description]

[Problems to be Solved by Invention]

An object of the present invention is to provide a novel protein capable of binding to human transferrin receptor.

In addition, another object of the present invention is to provide a novel protein capable of effectively presenting a disease antigen to dendritic cells.

Further, another object of the present invention is to provide a pharmaceutical composition for prevention or treatment of disease, which includes the novel protein described above.

Furthermore, another object of the present invention is to provide a method for treatment of a disease, which includes administering the novel protein described above.

[Means for Solving Problems]

According to an aspect of the present invention, there is provided a protein formed by self-assembly of ferritin monomers to which disease antigen epitopes are fused, wherein a binding force (K) to human transferrin receptor satisfies the following Equation 1:

[Equation 1]

$$K \leq 125 \ nM$$

(wherein K = [P][T]/[PT], wherein [P] represents a concentration of the protein in an equilibrium state of a binding reaction between the protein and the human transferrin receptor, [T] represents a concentration of the human transferrin receptor in the equilibrium state, and [PT] represents a concentration of a complex of the protein and the human transferrin receptor in the equilibrium state).
The protein of the present invention may have K ≤ 100 nM.
The protein of the present invention may have K ≤ 50 nM
The protein of the present invention may have K ≤ 30 nM
The protein of the present invention may have K ≤ 20 nM
In the present invention, the disease antigen epitope may be any one selected from the group consisting of gp100, MART-1, Melna-A, MAGE-A3, MAGE-C2, Mammaglobin-A, proteinsase-3, mucin-1, HPV E6, LMP2, PSMA, GD2, hTERT, PAP, ERG, NA17, ALK, GM3, EPhA2, NA17-A, TRP-1, TRP-2, NY-ESO-1, CEA, CA 125, AFP, Survivin, AH1, ras, G17DT, MUC1, Her-2/neu, E75, p53, PSA, HCG, PRAME, WT1, URLC10, VEGFR1, VEGFR2, E7, Tyrosinase peptide, B16F10, EL4 and neoantigens.
The ferritin monomer of the present invention may be derived from human ferritin heavy chains.
The protein of the present invention may have a spherical shape in which 24 ferritin monomers are self-assembled.
The disease antigen epitope of the present invention may be fused to at least one of sites between adjacent α-helixes of the ferritin monomer.
In the present invention, the disease antigen epitope may be fused at N-terminus or C-terminus of the ferritin monomer.
In the present invention, the disease antigen epitope may be fused to A-B loop, B-C loop, C-D loop or D-E loop of the ferritin monomer.
In the present invention, the disease antigen epitope may be fused between N-terminus and A helix or between E helix and C-terminus of the ferritin monomer.
In the present invention, the disease antigen epitope may be fused inside at least one of the helixes of the ferritin monomer.
The disease antigen epitope of the present invention may have an amino acid length of 25aa or less.
The protein of the present invention may include a water-soluble fraction, which is present in a ratio of 40% or more in an *E. coli* production system.
The disease antigen epitope of the present invention may be any one selected from the group consisting of brain cancer, head and neck cancer, bladder cancer, breast cancer, cervical cancer, colon cancer, colorectal cancer, endometrial cancer, esophageal cancer, leukemia, lung cancer, liver cancer, ovarian cancer, pancreatic cancer, prostate cancer, rectal cancer, kidney cancer, stomach cancer, testicular cancer, uterine cancer, vascular tumor, squamous cell carcinoma, adenocarcinoma, small cell carcinoma, melanoma, glioma, neuroblastoma, sarcoma, laryngeal cancer, parotid carcinoma, biliary tract cancer, thyroid cancer, actinic keratosis, acute lymphocytic leukemia, acute myeloid leukemia, adenocarcinoma, adenoma, glandular squamous cell carcinoma, anal duct cancer, anal cancer, anal rectal cancer, astrocytoma, large vaginal gland carcinoma, basal cell carcinoma, biliary cancer, bone cancer, bone marrow cancer, bronchial cancer, bronchial gland carcinoma, carcinoid, bile duct carcinoma, chronic lymphocytic leukemia, chronic myelogenous leukemia, clear cell carcinoma, connective tissue cancer, cyst adenoma, digestive system cancer, duodenal cancer, endocrine system cancer, endoderm sinus tumor, endometrial hyperplasia, endometrial adenocarcinoma, endothelial cell carcinoma, ventricular cell, epithelial cell cancer, orbital cancer, focal nodular hyperproliferation, gallbladder cancer, flank cancer, gastric basal cancer, gastrinoma, glioblastoma, glucagonoma, heart cancer, hemangioblastoma, hemangioendothelioma, hemangioma, hepatoadenoma, hepatic adenoma, hepatobiliary cancer, hepatocellular carcinoma, Hodgkin's disease, ileal cancer, insulinoma, intraepithelial neoplasm, intraepithelial squamous cell neoplasm, intrahepatic biliary cancer, invasive squamous cell carcinoma, jejunal cancer, joint cancer, pelvic cancer, giant cell carcinoma, colon cancer, lymphoma, malignant mesothelioma, mesothelioma, medullary epithelial carcinoma, meningeal cancer, mesothelial cancer, metastatic carcinoma, oral cancer, mucosal epithelial carcinoma, multiple myeloma, muscle cancer, nasal duct cancer, nervous system cancer, non-epithelial skin cancer, non-Hodgkin's lymphoma, chondrocyte carcinoma, oligodendrocyte cancer, oral cancer, osteosarcoma, papillary serous adenocarcinoma, penile cancer, pharyngeal cancer, pituitary tumor, plasmacytoma, sarcoma, pulmonary blastoma, rectal cancer, renal cell carcinoma, respiratory cancer, retinoblastoma, serous carcinoma, sinus cancer, skin cancer, small cell carcinoma, small intestine cancer, smooth muscle cancer, soft tissue cancer, somatostatin-secreting tumor, spinal cancer, squamous cell carcinoma, striatal muscle cancer, subcutaneous cell carcinoma, T cell leukemia, tongue cancer, ureteral cancer, urethral cancer, cervical cancer, uterine trunk cancer, vaginal cancer, VIPoma, genital cancer, hyperdifferentiated carcinoma and Wilm's tumor

In addition, according to another aspect of the present invention, there is provided a pharmaceutical composition for prevention or treatment of cancer, which includes the protein of the present invention.

The pharmaceutical composition of the present invention may be used for preventing or treating any one selected from the group consisting of melanoma, lung cancer, colon cancer, liver cancer, glioblastoma, ovarian cancer, colon cancer, head and neck cancer, bladder cancer, renal cell cancer, stomach cancer, breast cancer, metastatic cancer, prostate cancer, gallbladder cancer, pancreatic cancer and blood cancer.

The pharmaceutical composition of the present invention may be an injectable formulation.

The pharmaceutical composition of the present invention may be administered through intraperitoneal, intravenous, intramuscular, subcutaneous, intradermal, oral, topical, intranasal, pulmonary or rectal administration.

Further, according to another aspect of the present invention, there is provided a method for treatment of cancer, which includes administering the protein of the present invention to a subject.

According to the treatment method of the present invention, any one selected from the group consisting of melanoma, lung cancer, colon cancer, liver cancer, glioblastoma, ovarian cancer, colon cancer, head and neck cancer, bladder cancer, kidney cell cancer, stomach cancer, breast cancer, metastatic cancer, prostate cancer, gallbladder cancer, pancreatic cancer and blood cancer may be treated.

[Advantageous effects]

The protein of the present invention has excellent binding ability with human transferrin receptors.

The protein of the present invention provides a fused antigen epitope to an antigen-presenting cell to induce an immune action against the antigen.

The proteins of the present invention are capable of fusing antigen epitopes of various lengths at various positions.

The protein of the present invention has a substantially spherical shape by self-assembly of 24 ferritin monomers to which disease antigens are fused.

The protein of the present invention is a nanoparticle. This is significantly smaller in size than antibodies and the like.

The protein of the present invention can be easily produced through microorganisms such as *E. coli* and is obtained in a high ratio of soluble form.

The protein of the present invention can be used as an immune anticancer agent.

When a disease antigen is fused to the protein of the present invention, an immune response required for the treatment of the disease can be induced.

[Brief Description of Drawings]

FIG. 1A is a schematic diagram of an expression vector for producing a protein of the present invention in which a tumor antigen is expressed, and FIG. 1B illustrates a structure of the produced protein.

FIG. 2 is a schematic diagram illustrating a binding site of a tumor antigen and a transferrin receptor (TfR) on the surface of gp100-huHF nanoparticles prepared according to the present invention.

FIG. 3 illustrates TEM images and DLS results of the gp100-huHF protein of the present invention.

FIG. 4 illustrates results of measuring the binding affinity of the gp100-huHF protein of the present invention with the transferrin receptor (TfR).

FIG. 5 illustrates: a schematic diagram of an expression vector for preparing an immune checkpoint inhibitor (huHF-PD1 protein), into which a PD1 domain capable of binding to PD-L1 is inserted; a structure of the gp100-huHF protein; TEM image of the gp100-huHF protein of the present invention; a diameter distribution view of the gp100-huHF protein of the present invention; and results of measuring the binding affinity of huHF-PD1 protein with PD1 ligand (PD-L1), huHF-TPP1 (AB loop, CD loop) and αPD-L1 HCDR3 (CD loop, C-terminus), respectively.

FIG. 6 illustrates results of cellular uptake by dendritic cells of the protein of the present invention.

FIG. 7A illustrates results of comparing the efficiencies of huHF protein and huHF-PD1 protein to target cancer cells CT-26 and B16F10 through fluorescence images; FIG. 7B illustrates results of comparing the efficiencies of huHF protein and huHF-aPD-L1 HCDR3 (CD loop, C-terminus) to target CT-26 cells through fluorescence images; and FIG. 7C illustrates results of comparing the efficiencies of huHF protein, huHF-TPP1 and huHF-smPD1 to target CT-26 cells through fluorescence images.

FIG. 8 illustrates results of confirming the delivery efficiency of gp100-huHF to lymph nodes.

FIG. 9 illustrates results of comparing the cancer targeting efficiencies of huHF, PD-L1 antibody and huHF-PD1 protein to cancer cell CT-26, wherein the results of huHF, α-PD-L1 and PD1-huHF are shown from the left in the bar graph of relative fluorescence intensity for each organ.

FIG. 10 illustrates results of comparing the immunity efficiency to insertion site of gp100 in the gp100-huHF protein, wherein the left side of the bar graph for each group is the result obtained without gp100 and the right side is the result obtained with gp100.

FIG. 11A illustrates results of confirming whether OVA-huHF protein can increase OVA peptide antigen presentation of antigen-presenting cells through flow cytometry (FACS), while FIG. 11B illustrates results of determining the expression level of DC maturation marker of the protein wherein the results of MHC-II, CD80, CD40 and CD86 are shown from the left in the bar graph for each group shown in FIG. 11B.

FIG. 12 is a schematic diagram of an experimental method for confirming the tumor antigen inhibitory ability of gp100-huHF protein and a graph illustrating experimental results.

FIG. 13 is a schematic diagram of an experimental method for confirming the tumor formation inhibitory effect of huHF-PD1 protein in CT26 (colorectal cancer cells) and B16F10 (melanoma cells) in an animal model and graphs illustrating experimental results.

FIG. 14 illustrates a schematic diagram of an experimental method for confirming the tumor formation inhibitory effect in CT26 (colorectal cancer cells) and B16F10 (melanoma cells) due to effects of a combined treatment with huHF-PD1 protein, gp100-huHF and AH1-huHF protein in an animal model and graphs illustrating experimental results.

FIG. 15A illustrates results of comparing the T-cell mediated apoptosis efficiencies of PD-L1 antibody and huHF-PD1 protein in cancer cells CT26 and B16F10; FIG. 15B illustrates results of comparing the T-cell activity responses of PD-L1 antibody and huHF-PD1 protein in cancer cells CT26 and B16F10; and FIG. 15C illustrates T-cell activity responses to tumor antigens, respectively, by the combined treatment of AH1-huHF protein, gp100-huHF protein and huHF-PD1.

FIG. 16 illustrates results of confirming the induction of immune side effects with the existing antibody therapeutic agents.

FIG. 17 illustrates results of suppressing tumor recurrence in CT26 (colorectal cancer cells) by treatment using AH1-huHF protein and/or huHF-PD1 protein.

FIG. 18 illustrates results of extracting T cells from the body of the experimental mice and verifying the same in order to determine T-cell activity for inducing inhibition of tumor formation after tumor rechallenge of the huHF-PD1 protein, wherein the results of PBS, AH1-huHF, α-PD-L1, PD1-huHF, AH1-huHF + α-PD-L1 and AH1-huHF + PD1-huHF are shown in this order from the left side.

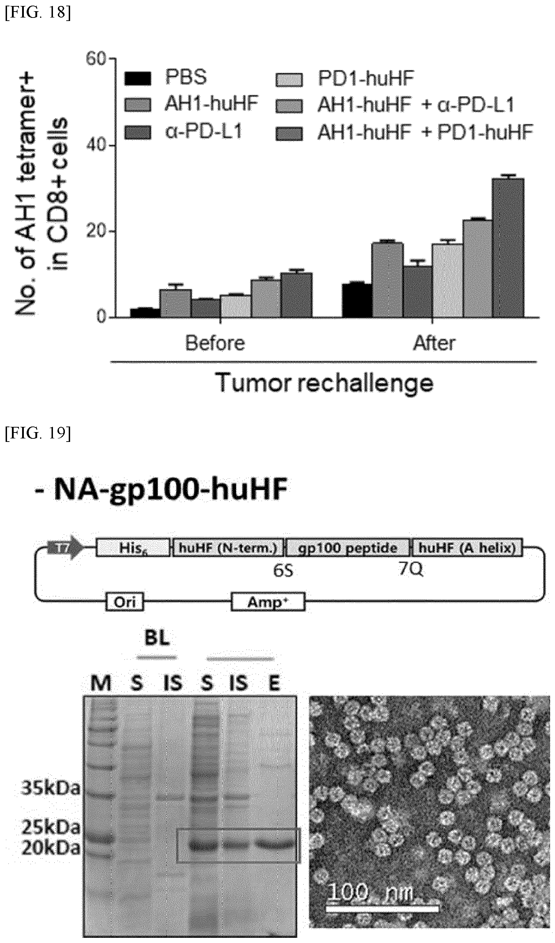

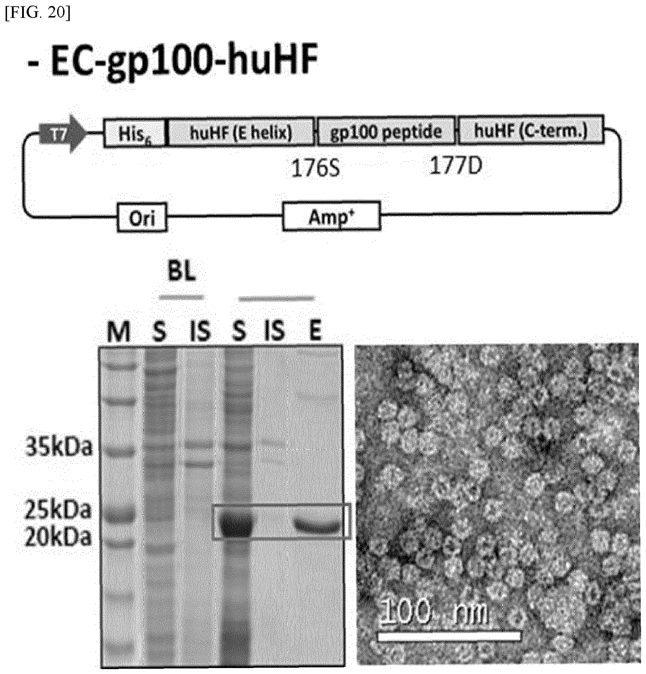

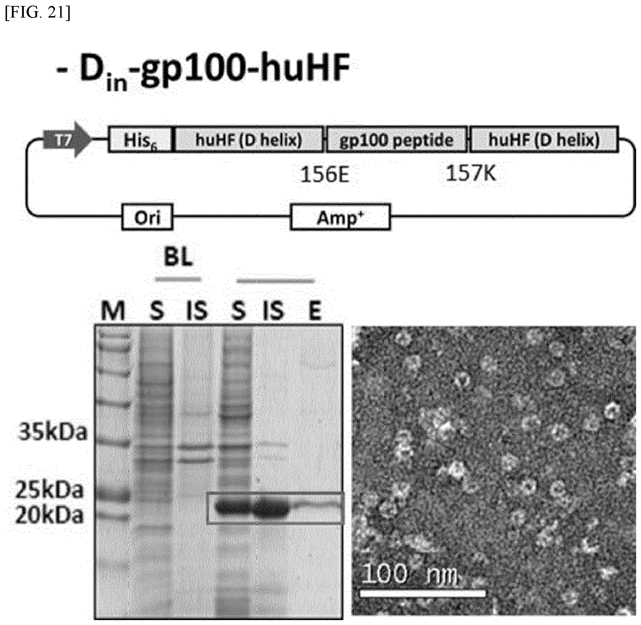

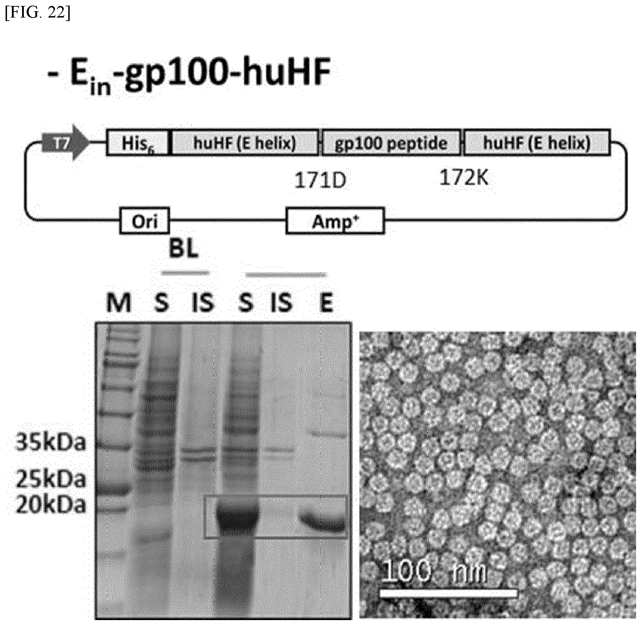

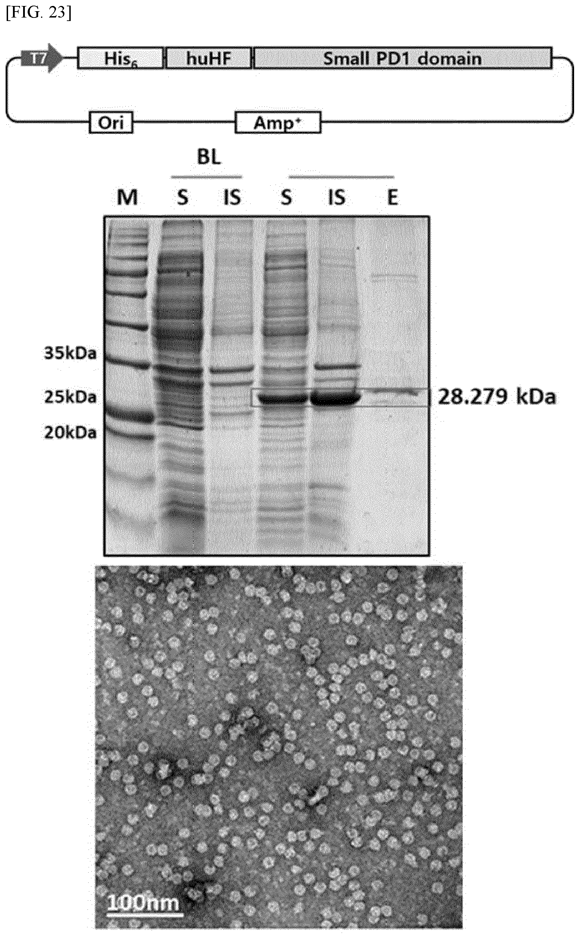

FIG. 19 illustrates a schematic diagram of a vector for preparation of NA-gp100-huHF and the production of the protein thereof; FIG. 20 illustrates a schematic diagram of a vector for preparation of EC-gp100-huHF and the production of the protein thereof; FIG. 21 illustrates a schematic diagram of a vector for preparation of D_{in}-gp100-huHF and the production of the protein thereof; FIG. 22 illustrates a schematic diagram of a vector for preparation of Ein0gp100-huHF and the production of the protein thereof, FIG. 23 is a vector schematic diagram for preparation of msmPD1-huHF and the production of the protein thereof.

FIG. 24 illustrates confirming the tumor inhibitory ability of PD1-huHF

FIG. 25 illustrates a schedule for assessment of the tumor inhibitory ability of the huHF-PD-L1-TIGIT dual blocker.

FIGS. 26 and 27 illustrate results of evaluating the tumor suppression ability of the huHF-PD-L1-TIGIT dual blocker.

FIGS. 28 and 29 illustrate results of evaluating the targeting ability of huHF-α-PD-L1 HCDR3 according to the binding site of the ferritin monomer.

FIG. 30 illustrates a schematic diagram of a vector of huHF-αPD-L1 HCDR3, and results of confirming the production and self-assembly of the protein thereof.

FIG. 31 illustrates a schematic diagram of a vector of huHF-aPD HCDR3, and results of confirming the production and self-assembly of the protein thereof.

FIG. 32 illustrates a schematic diagram of a vector of huHF-αCTLA4 HCDR3, and results of confirming the production and self-assembly of the protein thereof.

FIG. 33 illustrates a schematic diagram of a vector of huHF-aTIGIT HCDR3, and results of confirming the production and self-assembly of the protein thereof.

FIG. 34 illustrates a schematic diagram of a vector of huHF-αLAG3 HCDR3, and results of confirming the production and self-assembly of the protein thereof.

FIG. 35 illustrates a schematic diagram of a vector of huHF-αTIM3 HCDR3, and results of confirming the production and self-assembly of the protein thereof.

FIG. 36 illustrates a schematic diagram of a vector of huHF-αPD-L1-αTIGIT, and results of confirming the production and self-assembly of the protein thereof.

[Mode for Carrying out Invention]

The present invention relates to a protein that is formed by self-assembly of ferritin monomers to which disease antigen epitopes are fused, and is bound to a transferrin receptor.

The ferritin may be a ferritin derived from human, animals and microorganisms.

The human ferritin is composed of a heavy chain (21 kDa) and a light chain (19 kDa), and exhibits a feature of forming spherical nanoparticles through self-assembly ability of monomers constituting the ferritin. The ferritin may form a self-assembly having a spherical three-dimensional structure by gathering 24 monomers.

For human ferritin, an outer diameter may be about 12 nm and an inner diameter may be about 8 nm. The structure of the ferritin monomer may be a form in which five α-helix structures, namely A helix, B helix, C helix, D helix and E helix are sequentially linked, and may include an amorphous polypeptide moiety to link polypeptides each having α-helix structure, called a loop.

The loop is a region that is not structurally damaged even when a peptide or a small protein antigen is inserted into the ferritin. At this time, fusing a peptide to the loop via cloning may prepare a peptide-ferritin fused protein monomer in which a peptide such as an epitope is positioned on a monomer of the ferritin. A loop connecting A helix and B helix refers to A-B loop. Likewise, a loop connecting B helix and C helix is B-C loop, a loop connecting C helix and D helix is C-D loop, and a loop connecting D helix and E helix is D-E loop.

Information on ferritin is known from NCBI (GenBank Accession No. NM_000146, NM_002032, etc.).

Ferritin may be a ferritin heavy chain, specifically, a human ferritin heavy chain. The human ferritin heavy chain may be a protein represented by an amino acid sequence of SEQ ID NO: 1 derived from human. In the present specification, the ferritin may be used interchangeably with the "human ferritin heavy chain" or "huHF."

Disease antigens may be antigens of any disease that can be prevented, treated, alleviated or ameliorated by an immune response. For example, the disease antigen may be a cell surface antigen of a cancer cell, a pathogen cell, or a cell infected with a pathogen. A specific site to determine antigen specificity of a disease antigen refers to a disease antigen epitope.

The disease stated herein may be, for example, cancer or an infectious disease.

The cancer may be selected from the group consisting of, for example, brain cancer, head and neck cancer, bladder cancer, breast cancer, cervical cancer, colon cancer, colorectal cancer, endometrial cancer, esophageal cancer, leukemia, lung cancer, liver cancer, ovarian cancer, pancreatic cancer, prostate cancer, rectal cancer, kidney cancer, stomach cancer, testicular cancer, uterine cancer, vascular tumor, squamous cell carcinoma, adenocarcinoma, small cell carcinoma, melanoma, glioma, neuroblastoma, sarcoma, laryngeal cancer, parotid carcinoma, biliary tract cancer, thyroid cancer, actinic keratosis, acute lymphocytic leukemia, acute myeloid leukemia, adenocarcinoma, adenoma, glandular squamous cell carcinoma, anal duct cancer, anal cancer, anal rectal cancer, astrocytoma, large vaginal gland carcinoma, basal cell carcinoma, biliary cancer, bone cancer, bone marrow cancer, bronchial cancer, bronchial gland carcinoma, carcinoid, bile duct carcinoma, chronic lymphocytic leukemia, chronic myelogenous leukemia, clear cell carcinoma, connective tissue cancer, cyst adenoma, digestive system cancer, duodenal cancer, endocrine system cancer, endoderm sinus tumor, endometrial hyperplasia, endometrial adenocarcinoma, endothelial cell carcinoma, ventricular cell, epithelial cell cancer, orbital cancer, focal nodular hyperproliferation, gallbladder cancer, flank cancer, gastric basal cancer, gastrinoma, glioblastoma, glucagonoma, heart cancer, hemangioblastoma, hemangioendothelioma, hemangioma, hepatoadenoma, hepatic adenoma, hepatobiliary cancer, hepatocellular carcinoma, Hodgkin's disease, ileal cancer, insulinoma, intraepithelial neoplasm, intraepithelial squamous cell neoplasm, intrahepatic biliary cancer, invasive squamous cell carcinoma, jejunal cancer, joint cancer, pelvic cancer, giant cell carcinoma, colon cancer, lymphoma, malignant mesothelioma, mesothelioma, medullary epithelial carcinoma, meningeal cancer, mesothelial cancer, metastatic carcinoma, oral cancer, mucosal epithelial carcinoma, multiple myeloma, muscle cancer, nasal duct cancer, nervous system cancer, non-epithelial skin cancer, non-Hodgkin's lymphoma, chondrocyte carcinoma, oligodendrocyte cancer, oral cancer, osteosarcoma, papillary serous adenocarcinoma, penile cancer, pharyngeal cancer, pituitary tumor, plasmacytoma, sarcoma, pulmonary blastoma, rectal cancer, renal cell carcinoma, respiratory cancer, retinoblastoma, serous carcinoma, sinus cancer, skin cancer, small cell carcinoma, small intestine cancer, smooth muscle cancer, soft tissue cancer, somatostatin-secreting tumor, spinal cancer, squamous cell carcinoma, striatal muscle cancer, subcutaneous cell carcinoma, T cell leukemia, tongue cancer, ureteral cancer, urethral cancer, cervical cancer, uterine trunk cancer, vaginal cancer, VIPoma, genital cancer, hyperdifferentiated carcinoma and Wilm's tumor.

The infectious disease may be, for example, a viral, bacterial, fungal, parasitic or prion infection.

Cancer antigen epitopes may be gp100, MART-1, Melna-A, MAGE-A3, MAGE-C2, Mammaglobin-A, proteinsase-3, mucin-1, HPV E6, LMP2, PSMA, GD2, hTERT, PAP, ERG, NA17, ALK, GM3, EPhA2, NA17-A, TRP-1, TRP-2, NY-ESO-1, CEA, CA 125, AFP, Survivin, AH1, ras, G17DT, MUC1, Her-2/neu, E75, p53, PSA, HCG, PRAME, WT1, URLC10, VEGFR1, VEGFR2, E7, Tyrosinase peptide, B16F10, EL4 or neoantigens.

Neoantigen refers to an immunogenic peptide that is induced and formed by somatic mutations in tumor cells. The neoantigen forms a complex along with MHC I and migrates to the surface of a tumor cell, and thus may be displayed as an antigen epitope. T-cell receptors (TCRs) recognize the neoantigen-MHCI complex to trigger an immune response.

The disease antigen epitope is not limited to a specific length as long as it can be fused to the ferritin monomer.

The disease antigen epitope is not limited to a specific length as long as it does not interfere with self-assembly of the ferritin monomers.

The disease antigen epitope may be fused to any of the ferritin monomers. The disease antigen epitope is fused at a site that does not interfere with self-assembly of the ferritin monomer. The disease antigen epitope is preferably fused to the ferritin monomer such that it is exposed to the surface of the protein for the purpose of binding to the human transferrin receptor.

The disease antigen epitope may have an amino acid length of, for example, 25aa or less, 24aa or less, 23aa or less, 22aa or less, 21aa or less, 20aa or less, 19aa or less, 18aa or less, 17aa or less, 16aa or less, 15aa or less, 14aa or less, 13aa or less, 12aa or less, 11aa or less, 10aa or less, 9aa or less, 8aa or less, 7aa or less, 6aa or less, 5aa or less, etc.

The disease antigen epitope may have, for example, the amino acid length of 3aa or more, 4aa or more, 5aa or more, 6aa or more, 7aa or more, 8aa or more, 9aa or more, 10aa or more, etc.

Fusing the disease antigen epitope to the ferritin monomer may improve the binding ability of the protein, which was formed of self-assembled ferritin monomers, with the human transferrin receptor. Among constituent moieties of the ferritin monomer, a moiety incorporated into the monomer may protrude outward after binding of the disease antigen epitope.

The fusion site of the disease antigen epitope in the ferritin monomer is not limited to a specific position, but may include, for example, between adjacent a-helixes, N-terminus, C-terminus, A-B loop, B-C loop, C-D loop, D-E loop, between N-terminus and A helix, between E helix and C-terminus, and the inside of the helix or the like.

The disease antigen epitope may be fused at at least one of adjacent α-helixes. Further, the disease antigen epitope may be fused at the N-terminus or C-terminus of the ferritin monomer. Further, the disease antigen epitope may be fused to the A-B loop, B-C loop, C-D loop or D-E loop of the ferritin monomer. Further, the disease antigen epitope may be fused between the N-terminus and A helix of the ferritin monomer or between the E helix and C-terminus. Further, the disease antigen epitope may be fused to the inside of at least one of helixes of the ferritin monomers.

The protein of the present invention is composed of a self-assembly of ferritin monomers to which disease antigen epitopes are fused.

Ferritin is a self-assembled protein that forms an aggregate by forming an organizational structure or pattern on its own when several monomers are collected, and may form nanoscale proteins without additional manipulation.

The ferritin monomer to which the disease antigen epitope according to the present invention is fused may also form a self-assembled protein. For example, 24 ferritin monomers can be self-assembled to form spherical particles.

When the protein of the present invention forms particles, the particle may have a particle diameter of 8 to 50 nm, for example. Specifically, it may be 8 to 50 nm, 8 to 45 nm, 8 to 40 nm, 8 to 35 nm, 8 to 30 nm, 8 to 25 nm, 8 to 20 nm, 8 to 15 nm, etc., but it is not limited thereto.

The protein of the present invention has binding ability with a transferrin receptor (transferrin receptor 1, TfR) present on the surface of dendritic cells as antigen-presenting cells. Therefore, an antigen with such a fused antigen epitope fused antigen epitope is presented, and the immune system recognizes the antigen so that the immune response can be performed.

The protein of the present invention may have a binding force (or binding affinity; K) to human transferrin receptor which satisfies the following Equation 1:

$$[\text{Equation 1}]$$

$$K \leq 125 \text{ nM}$$

(wherein K = [P][T]/[PT], wherein [P] represents a concentration of the protein in an equilibrium state of a binding reaction between the protein and the human transferrin receptor, [T] represents a concentration of the human transferrin receptor in the equilibrium state, and [PT] represents a concentration of a complex of the protein and the human transferrin receptor in the equilibrium state).

With regard to the protein of the present invention, the binding force (K) to the human transferrin receptor may be 125 nM or less, 120 nM or less, 110 nM or less, 100 nM or less, 90 nM or less, 80 nM or less, 70 nM or less, 60 nM or less, 50 nM or less, 40 nM or less, 30 nM or less, 20 nM or less, 10 nM or less, etc. It means that the smaller the concentration in Equation 1, the higher the binding force to the human transferrin receptor.

With regard to the protein of the present invention, the binding force (K) to the human transferrin receptor may be 1 nM or more, 2 nM or more, 3 nM or more, 4 nM or more, or 5 nM or more.

The binding force (K) to the human transferrin receptor is measured in an equilibrium state of the binding reaction between the protein of the present invention and the human transferrin receptor. The concentration of the protein of the present invention ([P]), the concentration of the human transferrin receptor ([T]), and the concentration of a complex of the protein of the present invention and the human transferrin receptor ([PT]) in the equilibrium state may be measured by various known methods.

The binding force (K) to the human transferrin receptor may be measured according to, for example, a Microscale Thermophoresis (MST) method. An MST measuring device may be, for example, Monolith NT.115.

The concentration in Equation 1 may be obtained by utilizing the following Equations 2 and 3.

[Equation 2]

$$[PT] = 1/2 \times (([P_0]+[A_0]+[P][T]/[PT]) - (([P_0]+[T_0]+([P][T]/[PT])^2) - 4 \times [P_0] \times [T_0])^{1/2}).$$

(wherein [PT] represents a concentration of a complex of the protein and the human transferrin receptor in an equilibrium state of reaction, Po is an initial concentration of the protein, $T_0$ is an initial concentration of the human transferrin receptor, [P] represents a concentration of the protein in the equilibrium state of reaction, and [T] represents a concentration of the human transferrin receptor in the equilibrium state of reaction).

[Equation 3]

$$X= [PT]/[P_0]$$

(wherein [PT] represents a concentration of a complex of the protein and the human transferrin receptor in an equilibrium state of reaction, Po is an initial concentration of the protein, and X represents a ratio of protein that forms a complex together with the transferrin receptor in the protein).

The protein of the present invention may be produced in a microorganism to express a sequence encoding the protein.

As the microorganism, microorganisms known in the art may be used without limitation. For example, it may be *E. coli,* specifically BL21 (DE3), but it is not limited thereto.

In the case of producing a protein by a microbial system, the produced protein should be present in a dissolved state in the cytoplasm in order to facilitate separation/purification. In many cases, the produced protein exists in an aggregated state such as an inclusion body. The protein of the present invention has a high rate dissolved in the cytoplasm in the microbial production system. Accordingly, this is easy for separation/purification and use thereof.

The protein of the present invention may be produced, for example, in a state in which a water-soluble fraction ratio of the total protein is 40% or more in the *E. coli* system for producing the same. Specifically, the above ratio may be 40% or more, 50% or more, 60% or more, 70% or more, 80% or more, 90% or more, or 95% or more. The upper limit thereof may be, for example, 100%, 99%, 98%, 97%, 96% and the like.

The protein of the present invention may further include a linker peptide added between the human ferritin heavy chain protein and the disease antigen epitope. The linker peptide is not limited as long as it is a sequence for enhancing surface exposure of a protein by imparting flexibility to the epitope but may have, for example, an amino acid sequence of SEQ ID NO: 36 to SEQ ID NO: 38.

The linker peptide may have a length capable of securing an appropriate space between the disease antigen epitopes. For example, the linker peptide may be a peptide consisting of 1 to 20, 3 to 18, 4 to 15, or 8 to 12 amino acids. By adjusting the length and/or compositions of amino acids in the linker peptide, the spacing and orientation between the disease antigen epitopes may be regulated.

The present invention provides a pharmaceutical composition for prevention or treatment of cancer, which includes the proteins described above. All of the above descriptions in regard to the proteins may be applied as they are to the protein as an active ingredient of the pharmaceutical composition according to the present application.

The pharmaceutical composition of the present invention may include a pharmaceutically acceptable carrier. In the present invention, the term "pharmaceutically acceptable carrier" refers to a carrier or diluent that does not significantly irritate an organism and does not impair biological activity and properties of a component to be administered. The pharmaceutically acceptable carrier in the present invention may be used as one component or by mixing one or more of components, including saline, sterile water, Ringer's solution, buffered saline, dextrose solution, maltodextrin solution, glycerol or ethanol. Further, if necessary, other conventional additives such as antioxidants, buffers and bacteriostatic agents may be added and formulated in the form of an injection suitable for injecting into tissues or organs. Further, it may be formulated as an isotonic sterilization solution, or in some cases, a dry preparation (especially a freeze-dried preparation) that may form an injectable solution by adding sterile water or physiological saline thereto. Furthermore, a target organ-specific antibody or other ligands may be used in combination with the carrier so that it can specifically act on the target organ.

In addition, the composition of the present invention may further include a filler, an excipient, a disintegrant, a binder or a lubricant. Further, the composition of the present invention may be formulated using any method known in the art to allow rapid, sustained or delayed release of the active ingredient after administration to a mammal.

In one embodiment, the pharmaceutical composition may be an injectable formulation and may be administered intravenously, but it is not limited thereto.

In the present invention, the term "effective amount" means an amount necessary to delay the onset or progression of a specific disease to be treated or to entirely enhance the same.

In the present invention, the composition may be administered in a pharmaceutically effective amount. It is obvious to those skilled in the art that an appropriate total daily dose of the pharmaceutical composition may be determined by a practitioner or physician within the range of correct medical judgment.

For the purposes of the present invention, a specific pharmaceutically effective amount for a specific patient is preferably and differently applied depending upon type and extent of the reaction to be achieved, whether or not other agents are used occasionally, specific compositions, various factors such as an age, body weight, general health conditions, sex or diet of the patient, administration time, administration route and secretion rate of the composition, treatment period, drugs used with or concurrently with the specific composition, and similar factors well known in the medical field.

In the present invention, if necessary, the pharmaceutical composition may be accompanied by an instruction associated with the packaging in a form directed by a government agency in charge of the manufacture, use and sale of drugs, wherein the instruction represents approval of a private interest agency with respect to the form of a composition or administration to a human or animals, for example, the instruction may be a label approved by the US Food and Drug Administration for the prescription of drugs.

In addition to the above proteins, the pharmaceutical composition of the present invention may further include a ferritin protein ("an immune checkpoint inhibitor") to which molecules capable of binding to an immune checkpoint molecule are fused.

In order to remove cancer cells and perform immune response, T cells should recognize an antigen of the cancer cells present on an antigen-presenting cell thus to be activated. At this time, the immune checkpoint molecule has a role of being combined with T cells and thus serves to inactivate the same.

Such an immune checkpoint molecule may include, for example, Her-2/neu, VISTA, 4-1BBL, Galectin-9, Adenosine A2a receptor, CD80, CD86, ICOS, ICOSL, BTLA, OX-40L, CD155, BCL2, MYC, PP2A, BRD1, BRD2, BRD3, BRD4, BRDT, CBP, E2F1, MDM2, MDMX, PPP2CA, PPM1D, STAT3, IDH1, PD1, CTLA4, PD-L1, PD-L2, LAG3, TIM3, TIGIT, BTLA, SLAMF7, 4-1BB, OX-40, ICOS, GITR, ICAM-1, BAFFR, HVEM, LFA-1, LIGHT, NKG2C, SLAMF7, NKp80, LAIR1, 2B4, CD2, CD3, CD16, CD20, CD27, CD28, CD40L, CD48, CD52, EGFR family, AXL, CSF1R, DDR1, DDR2, EPH receptor family, FGFR family, VEGFR family, IGF1R, LTK, PDGFR family, RET, KIT, KRAS, NTRK1, NTRK2, etc.

The molecule capable of binding to the immune checkpoint molecule may include, for example, a ligand to the immune checkpoint molecule, or a fragment including a binding domain of the ligand for the immune checkpoint molecule.

The molecule capable of binding to the immune checkpoint molecule may be an antibody to the immune checkpoint molecule or an antigen binding fragment thereof.

A molecule capable of binding to an immune checkpoint molecule (abbrev. to "immune checkpoint molecule-binding molecule") is not limited to a specific length as long as it can be fused to a ferritin monomer. Molecules capable of binding to the immune checkpoint molecule are not limited to a specific length as long as the ferritin monomer does not interfere with self-assembly.

The molecules capable of binding to immune checkpoint molecules are preferably fused to ferritin monomers so as to be exposed to the protein surface for binding to human transferrin receptors.

The immune checkpoint molecule-binding molecule is fused to a ferritin monomer, and a fusion site thereof is not particularly limited to a specific position, but may include, for example, between adjacent α-helices, N-terminus, C-

terminus, A-B loop, B-C loop, C-D loop, D-E loop, between N-terminus and A helix, between E helix and C-terminus, and the inside of the helix or the like.

The immune checkpoint molecule-binding molecule may be fused to at least one of sites between adjacent α-helixes. Further, the immune checkpoint molecule-binding molecule may be fused at N-terminus or C-terminus of a ferritin monomer. Further, the immune checkpoint molecule-binding molecule may be fused to A-B loop, B-C loop, C-D loop or D-E loop of the ferritin monomer. Further, the immune checkpoint molecule-binding molecule may be fused between N-terminus and A helix or between E helix and C-terminus of the ferritin monomer. Further, the immune checkpoint molecule-binding molecule may be fused inside of at least one among helixes of the ferritin monomer.

An immune checkpoint inhibitor should be combined with the immune checkpoint molecule, and therefore, it preferably has a low binding force to a transferrin receptor. The transferrin receptor may be, for example, a human transferrin receptor but it is not limited thereto.

In order to reduce the binding force of the immune checkpoint inhibitor to the transferrin receptor, the immune checkpoint molecule-binding molecule may be fused at a site involved in binding of ferritin to the transferrin receptor.

Further, in a ferritin protein to which the immune checkpoint molecule-binding molecule is fused, the ferritin protein may have a mutated site involved in binding to the transferrin receptor.

There is a site involved in binding to the transferrin receptor in the ferritin monomer, and the ferritin monomer may have a corresponding site mutated to decrease the binding force to the transferrin receptor.

As a specific example of the case of using the sequence of SEQ ID NO: 1, the amino acid selected from the group consisting of 14, 15, 22, 81 and 83 in the sequence of SEQ ID NO: 1 may have been substituted with another amino acid. The amino acid to be substituted may be, for example, alanine, glycine, valine, leucine, etc., but it is not limited thereto.

The present invention provides a method for treatment of cancer, which includes administering the above protein. All of the descriptions in regard to the above protein may be applied as they are to the protein as an active ingredient in the cancer treatment method according to the present application.

The treatment method of the present invention may include administering the above protein to a subject suffering from cancer.

The subject suffering from cancer may be an animal with cancer, specifically a mammal with cancer, and more specifically may be a human suffering from cancer.

The protein may be administered in a therapeutically effective amount.

In the present invention, the term "administration" means introducing the composition of the present invention to a patient by any suitable method, and a route of administration of the composition of the present invention may include various routes, either oral or parenteral, as long as it can reach the target tissue. Intraperitoneal administration, intravenous administration, intramuscular administration, subcutaneous administration, intradermal administration, oral administration, topical administration, intranasal administration, intrapulmonary administration, or rectal administration may be implemented, but they are not limited thereto.

The method of the present invention may further include administering a ferritin protein, to which the immune checkpoint molecule-binding molecule is fused, to the subject described above.

The immune checkpoint molecule and the molecule capable of binding thereto may be within the above described range, but they are not limited thereto.

The ferritin protein fused with the immune checkpoint molecule-binding molecule may be administered simultaneously or sequentially along with a protein formed by self-assembly of ferritin monomers to which a disease antigen epitope is fused.

In the case of sequential administration, the order of administration is not limited, and the above ferritin protein may be administered before or after administration of the protein formed by self-assembly of ferritin monomers to which the disease antigen epitope is fused.

Hereinafter, examples will be described in detail in order to describe the present invention in detail.

## EXAMPLES

### 1. Preparation of expression vector for synthesis of candidate protein

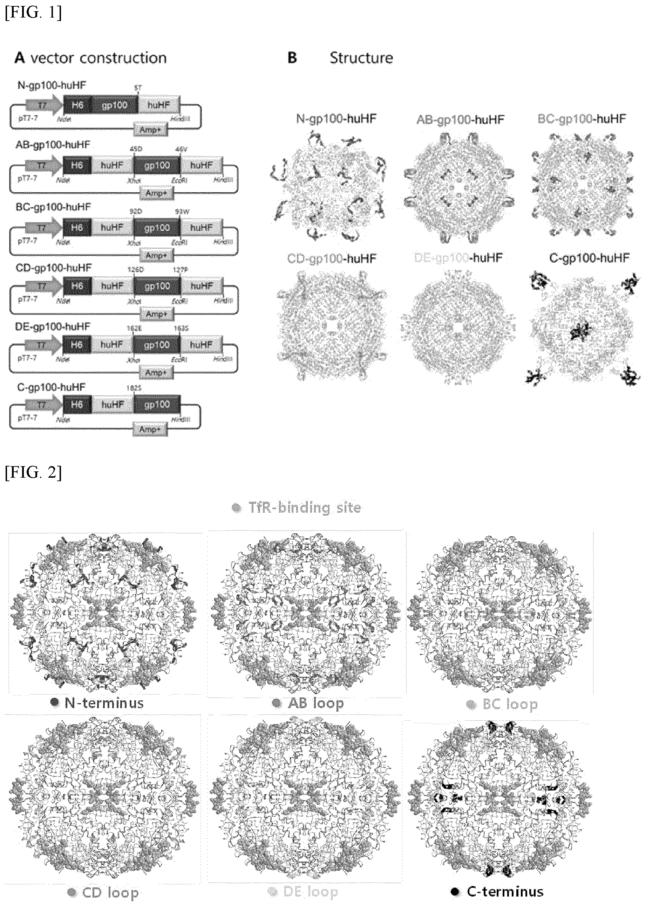

huHF is a spherical protein (12 nm) composed of 24 monomers, wherein each monomer is composed of a total of five (5) α-helixes. The present inventors have acquired a delivery system, in which gp100 peptide was inserted at various sites of huHF, by inserting the gp100 peptide as one of actual tumor antigens at a loop between α-helixes of huHF monomer (AB loop among huHF 5T to 176G; between 45D/46V, BC loop; 92D/93W, CD loop; 126D/127P, DE loop; 162E/163S, based on PDB 3AJO sequence), N-terminus and/or C-terminus through gene cloning (FIGS. 1 and 2). Through the previous study (U.S. Patent No. 10,206,987), the present inventors have selected the surface construction of huHF nanoparticles with the best surveillance lymph node targeting efficiency as cancer-specific antigen delivery nanoparticles.

In this regard, the candidate proteins of Table 1 below were subjected to PCR according to the vector schematic diagram of Table 2 below, such that proteins huHF, huHF-gp100 (SEQ ID NO: 2; melanoma specific antigen), OVA (SEQ ID NO: 3), AH1 (SEQ ID NO: 4) (AB; 45D/46V, BC; 92D/93W, CD; 126D/127P, DE; 162E/163S, N-terminus, C-terminus), huHF-PD1 (SEQ ID NO: 5; active site of PD1 domain), huHF-TPP1 (SEQ ID NO: 6) (AB, CD loop), huHF-αPD-L1 HCDR3 (SEQ ID NO: 7) (CD loop, C-terminus) and huHF-smPD1 (SEQ ID NO: 8) particles were prepared. At this time, the OVA was used as an immunospecific antigen. Likewise, AH1 was used as a tumor specific antigen of colorectal cancer cells, and gp100 was used as a tumor specific antigen of melanoma cells. All the prepared plasmid expression vectors were purified on an agarose gel, followed by confirming a sequence thereof through complete DNA sequencing.

Specifically, PCR products required for preparation of each expression vector were sequentially inserted into the plasmid pT7-7 vector using the primer set in Table 3 below, so as to construct an expression vector capable of expressing each protein. In this case, linker peptides of Table 4 below could be further included.

[TABLE 1]

| Sequence No. | Candidate protein |
|---|---|
| 2 | Gp100 |
| 3 | Ovalbumin |
| 4 | AH1 |
| 5 | PD1 |
| 6 | TPP1 |
| 7 | αPD-L1 HCDR3 |
| 8 | smPD1 (small PD1 domain) |
| 9 | RFP |

[TABLE 2]

| Protein | Expression vector |
|---|---|
| huHF | NH2-NdeI-(His)6-huHF-HindIII-COOH |
| [gp100/AH1/OVA ]-huHF loops | AB(45D/46V), BC(92D/93W), CD(126D/127P), DE(162E/163S):NH2-NdeI-(His)6-huHF-[gp100(KVPRNQDWL)/OVA(SIINFEKL)/AH1(SPSYVYHQF)] -huHF-HindIII-COOH N-terminus: NH2-NdeI-(His)6-[gp 100(KVPRNQDWL)/OVA(SIINFEKL)/AH1 (SPSYVYHQF)]-huHF -HindIII-COOH C-terminus: NH2-NdeI-(His)6-huHF-[gp 100 (KVPRNQDWL)/OVA(SIINFEKL)/AH1(SPSYVYHQF)]-HindIII-COOH Between N-terminus and A helix (6S/7Q), the middle of D helix (156R/157K), the middle of E helix (173H/174T), between E helix and C-terminus (178S/179D)NH2-NdeI-(His)6-[gp100 (KVPRNQDWL)]-huHF-HindIII-COOH |
| huHF-PD1 | NH2-NdeI-huHF-PD1(22-170)-HindIII-COOH |
| huHF-TPP1 (AB, CD loop) | NH2-NdeI-huHF-linker-TPP1-linker-HindIII-COOH |
| huHF-αPD-L1 HCDR3 (CD loop, C-terminus) | NH2-NdeI-huHF-αPD-L1 HCDR3-HindIII-COOH |
| huHF-smPD1 | NH2-NdeI-huHF-smPD1-HindIII-COOH |
| huHF-RFP | NH2-NdeI-(His)6-huHF-Xho1-linker(G3SG3TG3SG3T)-RFP-HindIII-COOH |

[TABLE 3]

| Sequence No. | Designation | Insertion site |
|---|---|---|
| 10 | N-gp100_F | N-terminus |
| 11 | N-gp100_R | |
| 12 | AB-gp100_F | AB loop |
| 13 | AB-gp100_R | |
| 14 | BC-gp100_F | BC loop |
| 15 | BC-gp100_R | |
| 16 | CD-gp100_F | CD loop |
| 17 | CD-gp100_R | |
| 18 | DE-gp100_F | DE loop |
| 19 | DE-gp100_R1 | |
| 20 | DE-gp100_R2 | |
| 21 | DE-gp100_R3 | |
| 22 | C-gp100_F | C-terminus |
| 23 | C-gp100_R | |
| 24 | NA-gp100_F_1 | Between N-terminus and A-helix |
| 25 | NA-gp100_F_2 | |
| 26 | NA-gp100_F_3 | |
| 27 | NA-gp100_R | |
| 28 | intD-gp100_F | The middle of D helix |
| 29 | intD-gp100_R_1 | |
| 30 | intD-gp100_R_2 | |
| 31 | intE-gp100_F | The middle of E helix |
| 32 | intE-gp100_R_1 | |
| 33 | intE-gp100_R_2 | |
| 34 | EC-gp100_F | Between E-helix and C-terminus |
| 35 | EC-gp100_R | |

[TABLE 4]

| Sequence No. | Designation |
|---|---|
| 36 | Linker 1 |
| 37 | Linker2 |
| 38 | Linker3 |

## 2. Biosynthesis of candidate proteins

*E. coli* strain BL21(DE3)[F-ompThsdSB(rB-mB-)] was transformed with the above-prepared expression vector, respectively, and ampicillin-resistant transformants were selected. The transformed *E. coli* was cultured in a flask (250 mL Erlenmeyer flasks, 37 °C, 150 rpm) containing 50 mL of Luria-Bertani (LB) medium (containing 100 mg L-1 ampicillin). When a medium turbidity (O.D600) reached about 0.5-0.7, isopropyl-β-dithiogalactopyranosid (IPTG) (1.0 mM) was injected to induce expression of the recombinant gene.

After incubating at 20 °C for 16-18 hours, the cultured *E. coli* was centrifuged at 4,500 rpm for 10 minutes to recover a cell precipitate, followed by suspending the precipitate in 5 ml of a disruption solution (10 mM Tris-HCl buffer, pH 7.5, 10 mM EDTA), and then crushing the same using an ultrasonic crusher (Branson Ultrasonics Corp., Danbury, CT, USA). After crushing, centrifugation was performed at 13,000 rpm for 10 minutes, and the supernatant and insoluble aggregates were separated. The separated supernatant was used for later experiments.

**3. Purification of candidate protein and adhesion of fluorescent substance**

The supernatant obtained in Example 2 was purified through the following three-step process. First, 1) $Ni^{2+}$-NTA affinity chromatography using a combination of nickel and histidine fused to the recombinant protein was conducted, then 2) the recombinant protein was concentrated and a fluorescent substance was adhered through buffer exchange, and lastly, 3) sucrose gradient ultracentrifugation was implemented to separate only the adhered self-assembled protein. Detailed description of each step is as follows.

1) $Ni^{2+}$-NTA affinity chromatography

To purify the recombinant protein, the cultured *E. coli* was recovered in the same manner as specified above, and the cell pellets were resuspended in 5 mL lysis buffer (pH 8.0, 50 mM sodium phosphate, 300 mM NaCl, 20 mM imidazole), followed by crushing the cells using an ultrasonic crusher. The crushed cell solution was centrifuged at 13,000 rpm for 10 minutes to separate only the supernatant, and then each recombinant protein was separated using a $Ni^{2+}$-NTA column (Qiagen, Hilden, Germany) (washing buffer: pH 8.0, 50 mM sodium phosphate, 300 mM NaCl, 80 mM imidazole/elution buffer: pH 8.0, 50 mM sodium phosphate, 300 mM NaCl, 200 mM imidazole).

2) Concentration, buffer exchange, and fluorescent substance adhering process

For imaging, huHF-gp100 particles and huHF-PD1 particles were placed on a column, and 3 ml of recombinant protein eluted through $Ni^{2+}$-NTA affinity chromatography was put in an ultra centrifugal filter (Amicon Ultra 100K, Millipore, Billerica, MA), followed by centrifugation thereof in the column at 5,000 g until 1 ml of the solution remained. Thereafter, in order to adhere NIR fluorescent substances cy5.5 and fluorescein isothiocyanate (FITC), the protein particles were subjected to buffer change with sodium bicarbonate (0.1 M, pH 8.5) buffer, followed by adhering the fluorescent substances at room temperature for 12 hours.

3) Sucrose gradient ultra-centrifugation

Sucrose was added to PBS (2.7 mM KCl, 137 mM NaCl, 2 mM KH2PO4, 10 mM Na2HPO4, pH 7.4) buffer by concentration to prepare solutions containing 40%, 35%, 30%, 25%, 20% sucrose, respectively. Then, the sucrose solutions with different concentrations (45 to 20%) were added by 2 ml to an ultra-centrifugation tube (ultraclear 13.2 ml tube, Beckman) in the order of the concentrations starting from the highest concentration solution, followed by filling the tube with 1 ml of recombinant protein solution present in the prepared buffer for self-assembly, and then carrying out ultra-centrifugation at 35,000 rpm for 16 hours at 4 °C (Ultracentrifuge L-90k, Beckman). After centrifugation, the upper layer (20-25% sucrose solution portion) was carefully pipetted to replace the buffer of the recombinant protein using an ultra centrifugal filter and PBS buffer, as specified in the above item 2).

**4. Verification of assembly of protein particles**

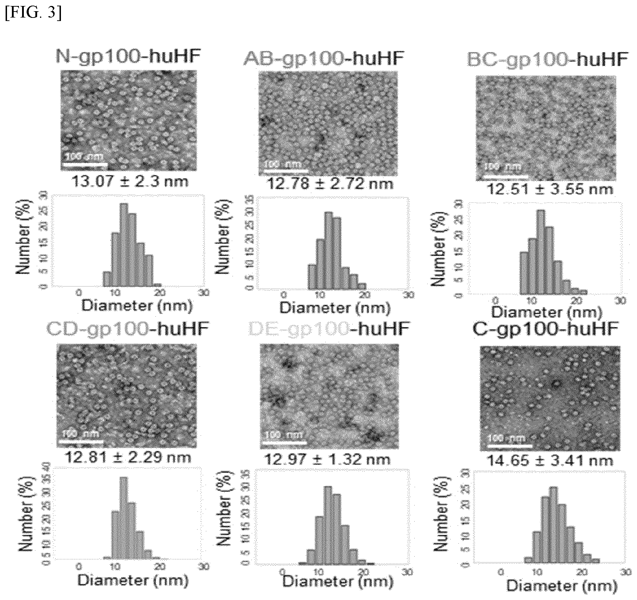

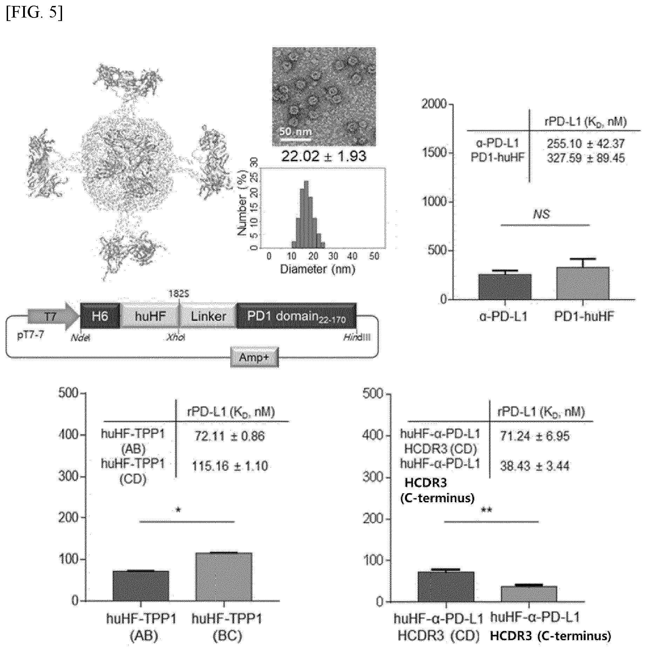

For structural analysis of purified recombinant protein for each of the proteins produced in Example 3 (gp100-huHF-loops, huHF-PD1, huHF-TPP1, huHF-αPD-L1 HCDR3, huHF-smPD1), the recombinant protein was photographed by transmission electron microscopy (TEM). First, an undyed purified protein sample was placed on a carbon-coated copper electron microscope grid and then naturally dried. To obtain stained images of the proteins, electron microscopy grids containing naturally dried samples were incubated with 2% (w/v) aqueous uranyl acetate solution at room temperature for 10 minutes, and then washed 3-4 times with distilled water. As a result of observing the protein image using a Philips Technai 120 kV electron microscope, it was confirmed that each of the particles has formed spherical nanoparticles (FIGS. 3 and 5). Further, through dynamic light scattering (DLS) measurement, each of gp100-huHF-loops, huHF-PD1, huHF-TPP1 (AB, CD loops), huHF-αPD-L1 HCDR3 (CD loop, C-terminus) and huHF-smPD1 particles was subjected to measurement of particle diameter in the solution (FIGS. 3 and 5).

**5. Determination of binding ability between OVA-huHF-loops protein and TfR, and binding ability between huHF-PD1, huHF-TPP1 (AB, CD loops), huHF-αPD-L1 HCDR3 (CD loop, C-terminus) or huHF-smPD1 protein and PD-L1**

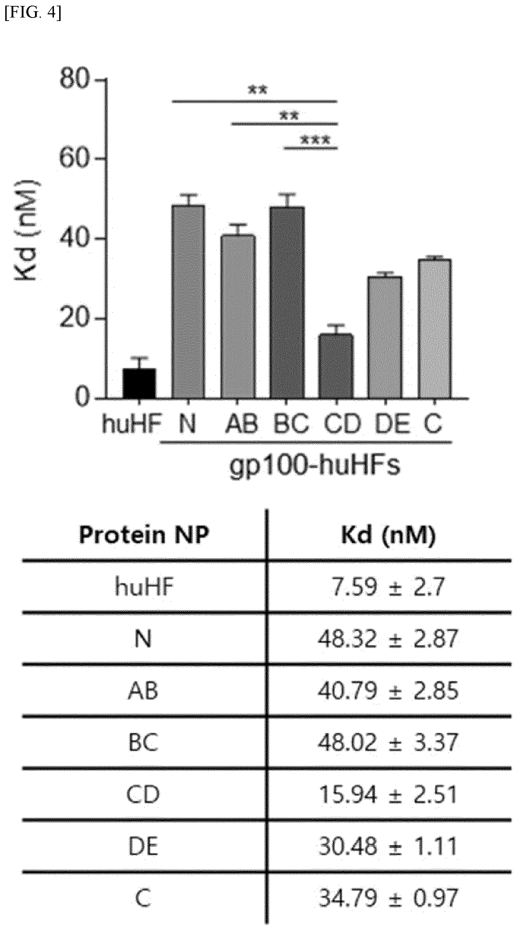

In order to prove the efficacy of the huHF transporter to increase immune cell activity, the present research team has determined the binding ability of the purified recombinant protein of each protein (gp100-huHF-loops) produced in Example 3 with the transferrin receptor (TfR) by means of Microscale Thermophoresis (MST) equipment. As a result, it was confirmed that huHF nanoparticles containing no tumor antigen had the most excellent binding ability with TfR, and the binding ability of CD-loop-gp100 nanoparticles with a tumor antigen inserted between CD helixes was second to the best. From the above results, it was indirectly confirmed that the CD-loop-gp100 particles were the best in terms of not interfering with the binding to TfR (FIG. 4).

Programmed cell death protein 1 (PD-1) is a protein on the surface of T-cells and binds to PD-L1, which is expressed on the surface of cancer cells, thereby inducing a decrease in T-cell activity. Therefore, when inhibition of the binding of PD-1 and PD-L1 in T cells is induced using a protein, in which a binding site of PD-1 to bind to PD-L1 expressed on the surface of cancer cell was exposed on the surface, T-cell activity inhibition is reduced whereby it could be expected to increase the efficiency of anticancer immunotherapy. Since it was determined that, rather than exposing active sites of PD-1 and CTLA-4 antibodies, which were intended to develop by the present inventors, on the protein surface, it is more efficient to induce self-assembly of the protein by exposing the binding site of PD-1 that binds to PD-L1 ("PD-L1 binding site of PD-1") on the protein surface in terms of protein expression, the PD-L1 binding site of PD-1 was synthesized in huHF (the binding active site 22G-170V in the PD-1 sequence, PD-L1 targeting peptide TPP1, HCDR3 sequence of PD-L1 antibody, the binding active site of PD-L1 (small PD1 domain)).

After gene cloning of the nanoparticles was conducted, it was expressed thus to induce particle synthesis through protein self-assembly. This was confirmed through TEM images. Further, in order to confirm whether the actually synthesized huHF-PD1 protein actually binds to the PD-1 ligand (PD-L1), the binding ability (that is, binding affinity; Kd) of the huHF-PD1 protein produced in Example 3 to PD-L1 was measured using an ELISA technique. After binding the recombinant PDL1 protein at a concentration of 2 ug/ml to a 96-well plate for 16-18 hours, the binding affinity of huHF-PD1 protein as well as PD-L1 antibody which is currently used immune antibody therapeutic agents, to PD-L1, respectively, was calculated with Langmuir equation.

As a result of measuring the binding affinity, Kd value of huHF-PD1 to the recombinant protein PD-L1 was measured to be 327.59 nM, which is higher than 770 nM that is a literature value of PD1-PDL1 binding affinity. Further, the above Kd value was similar to Kd value of PD-L1 and PD-L1 antibody, that is, 255.10 nM. From the above results, it was confirmed that the protein produced by exposing a PD-1 binding domain on the surface of huHF surface has the binding ability with PD-L1 (FIG. 5).

Further, the binding affinity between the actually synthesized huHF-αPD-L1 HCDR3 (CD loop, C-terminus) protein and PD-L1 was also measured by ELISA technique. From the measurement, the binding affinity of the huHF-αPD-L1 HCDR3 (CD loop) particles was 71.24 nM and the binding affinity of huHF-αPD-L1 HCDR3 (C-terminus) particles was 38.43 nM, respectively, thereby confirming that these proteins also have the binding ability with PD-L1 (FIG. 5).

Additionally, in order to confirm whether the actually synthesized huHF-TPP1 (AB, CD loops) protein actually binds to the PD-1 ligand (PD-L1), the binding affinity (Kd) of the huHF-TPP1 protein produced in Example 3 to PD-L1 was measured by the Microscale Thermophoresis (MST) equipment.

As a result of the measurement, the Kd value of huHF-TPP1 (AB loop) to PD-L1 was 72.105 nM, the Kd value of huHF-TPP1 (CD loop) to PD-L1 was 115.16 nM, the Kd value of huHF-αPD-L1 HCDR3 (CD loop) was 71.24 nM, and the Kd value of huHF-αPD-L1 HCDR3 (C-terminus) was 38.43 nM (FIG. 5).

**6. Dendritic cell uptake experiment of gp100-huHF-loops protein, and verification of colorectal cancer cell-targeting ability of huHF, huHF-PD1, huHF-TPP1 (AB, CD loops), huHF-aPD-Ll HCDR3 (CD loop, C-terminus), huHF-smPD1 PDL1 antibody therapeutic agents**

Dendritic cell uptake efficiencies of the huHF protein as well as the gp100-huHF-loops proteins produced in Example 3, to which the fluorescent substance was adhered, were compared.

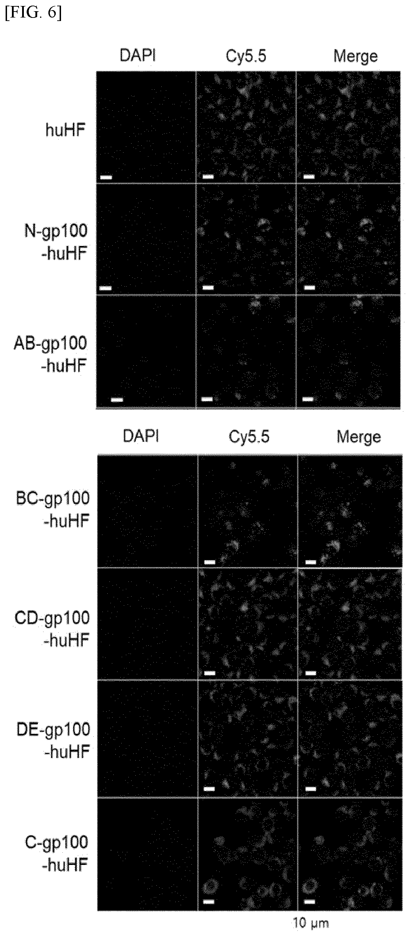

After each nanoparticle was reacted with the dendritic cells at 300 nM for 30 minutes, a fluorescence signal was measured through a confocal device (LSM 700). In this regard, it was confirmed that the binding ability of the CD-loop-gp100 protein with the tumor antigen inserted between the CD helixes was superior next to that of the protein of huHF itself. From the above results, it was also indirectly confirmed that the CD-loop-gp100 protein was the best in terms of not interfering with the binding to TfR (FIG. 6).

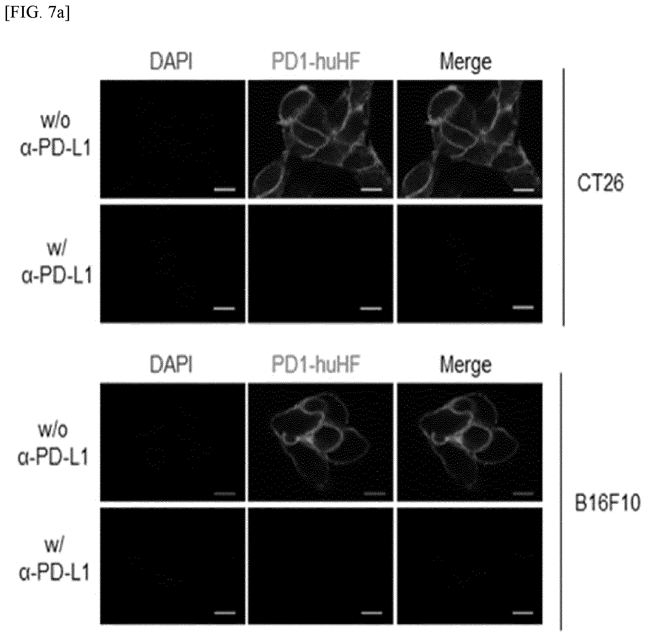

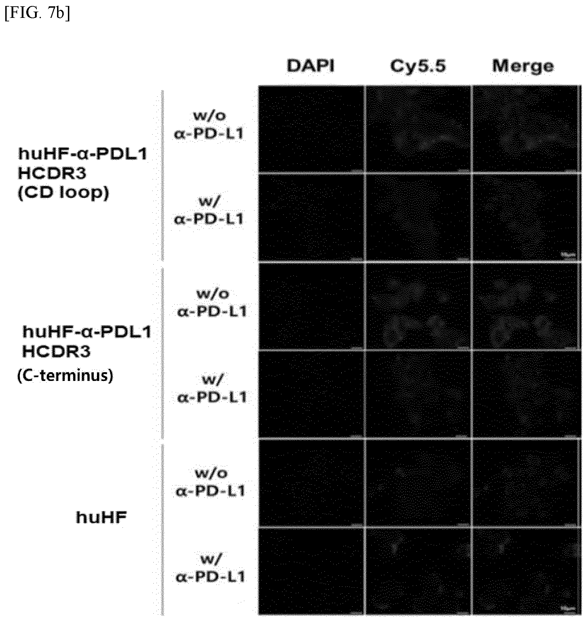

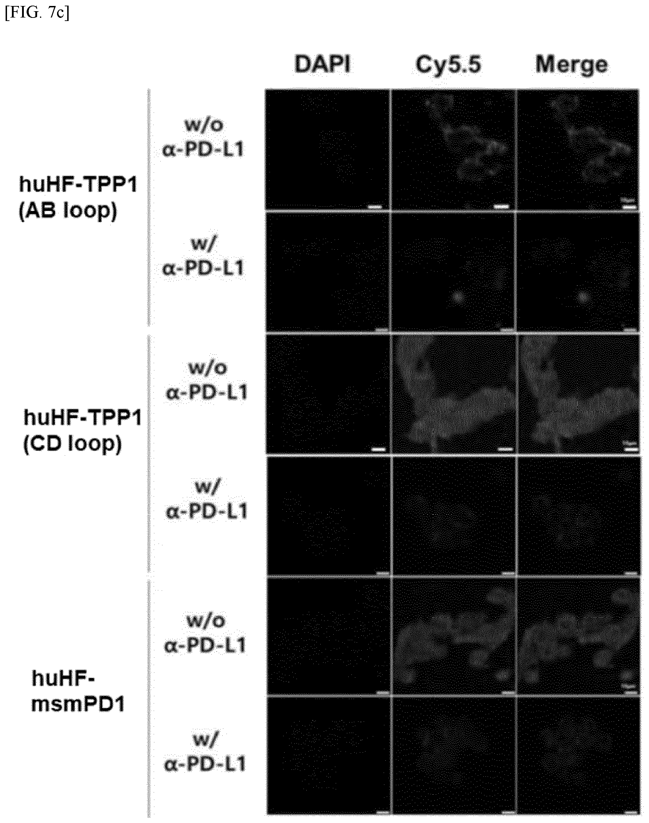

In order to compare CT26 colorectal cancer and B16F10 melanoma targeting efficiencies of the huHF, huHF-PD1, huHF-TPP1 (AB, CD loops), huHF-αPD-L1 HCDR3 (CD loops, C-terminus) and huHF-smPD1 proteins produced in Example 3, respectively, to which the fluorescent substance was adhered, CT26 colorectal cancer cells and B16F10 melanoma cells were reacted with each protein at a concentration of 300 nM, followed by comparing fluorescence signals to confirm cell uptake efficiency. As a result, as shown in FIGS. 7A to 7C, it was confirmed that each of huHF-PD1 (FIG. 7A), huHF-αPD-L1 HCDR3 (CD loops, C-terminus) (FIG. 7B), huHF-TPP1 (AB, CD loops) (FIG. 7C) and huHF-smPD1 (FIG. 7C) proteins was bound to cancer cells and exhibited a florescent signal rather than the control, huHF protein. Further, after treatment with PD-L1 antibody capable of masking PD-L1 expressed on the surface of the cancer cells for 20 minutes, when the huHF protein, huHF-PD1 protein, huHF-aPD-L1 HCDR3 protein and huHF-smPD1 protein were reacted respectively, it was confirmed that neither was combined.

## 7. NIR image analysis using the prepared proteins

Based on the above experimental results, after adjusting the fluorescence intensities of the five (5) proteins produced in Example 3 and then injecting the same into 5-week-old nude mice (n=3 per each experimental group), the gp100 antigen-expressing tumor was injected subcutaneously (foot pad injection), followed by analyzing a degree of tumor growth for a predetermined period of time to investigate whether all of the huHF-gp100 loop proteins had good targeting efficiency in lymph nodes. Each particle was injected into the right foot of a mouse by 20 μl, and the experiment was conducted for 1 hour.

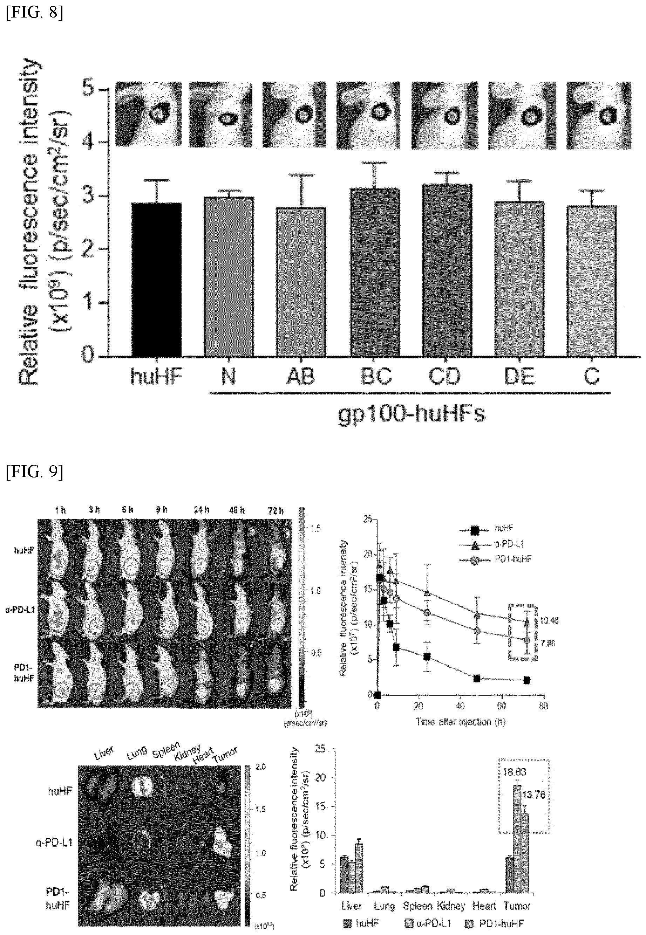

As a result, as shown in FIG. 8, when a cancer-specific antigen peptide was inserted into each of the AB loop, BC loop, CD loop, DE loop, N-terminus and C-terminus, respectively, it was confirmed that nanoparticle delivery efficiency to the lymph nodes in all proteins is good. Further, it was confirmed that tumor growth inhibitory effects are highest in the group injected with gp100-huHF (126 loop) nanoparticles that improved highest the activity of cancer antigen-specific immune cells.

Further, in order to confirm whether the huHF-PD1 protein binds to PD-L1 exposed on the surface of the actual tumor cells, the huHF protein and huHF-PD1 protein, respectively, to which a cy5.5 fluorescent substance is adhered, were injected in mice with growing CT-26 colorectal cancer cells, followed by comparing the cancer targeting efficiency. At this time, the PD-L1 antibody therapeutic agent that is actually used in clinical practice was used as a control. For 2 days after injection into mice, a particle targeting pattern in the body was observed with a Cy5.5 bandpass emission filter and a special C-mount lens or an IVIS spectrum imaging system (Caliper Life Sciences, Hopkinton, MA) (FIG. 9; in the lower graph at the right side, Y-axis represents a retention time in the body).

As a result, as shown in FIG. 9, it could be seen that the huHF-PD1 protein had better cancer cell targeting efficiency than the control huHF protein. However, in the above results, although the actual antibody therapeutic agent showed better cancer targeting efficiency and retention time in the body than the huHF-PD1 protein, this is a result obtained since the *in vivo* retention time of the antibody therapeutic agent is too long, which is directly related to the problem of *in vivo* immune side effects. Accordingly, it was confirmed that the protein of the present invention has advantages in both side effects and influences of the side effects.

## 8. Experiment to confirm secretion of specific cytokines through CD 8+ T cell assay

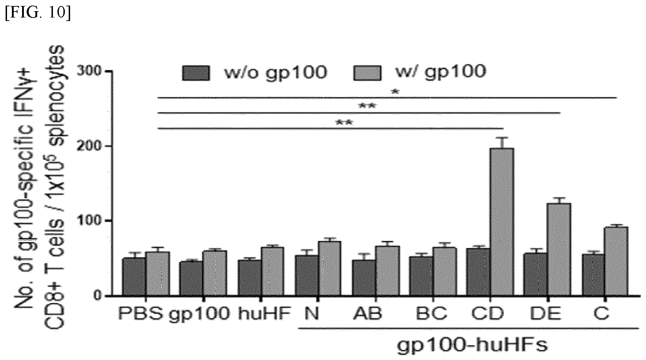

PBS (buffer) and huHF-gp100 loops protein were prepared by the methods of Examples 1 to 3, followed by boosting the immune response of the immune cells in the lymph nodes through vaccine injection into C57BL/6 mice once a week for a total of 3 weeks. Then, the spleen where the immune cells gathered was excised from each mouse and pulverized. Next, after extracting CD8+ T-cells in which the immune response was specifically induced by gp100 melanoma-specific antigen in the pulverized spleen, the T-cells were reacted with a specific partial antigen peptide of gp100 (KVPRNQDWL), which is known to induce an immune response *in vitro,* followed by investigating whether gp100-specific cytokines were secreted through FACS assay. As a result, it was confirmed that CD8+ T-cells extracted from the spleen of the mouse injected with 126-gp100-huHF protein have secreted the most cytokine (FIG. 10).

## 9. Confirmation of MHC-OVA presentation and verification experiment of costimulatory effector expression on dendritic cell surface

PBS (buffer) and huHF-OVA loops protein were prepared by the methods of Examples 1 to 3, followed by boosting the immune response of the immune cells in the lymph nodes through vaccine injection into C57BL/6 mice once a week for a total of 3 weeks. Then, the spleen where the immune cells gathered was excised from each mouse and pulverized. Next, OVA immune peptide in the pulverized spleen was used to identify a protein that best exposes the peptide on the surface of the dendritic cells (DCs) using an antibody that captures the surface-exposed dendritic cells through MHC-I.

As a result, it was confirmed that the nanoparticles containing the OVA peptide in the CD-loop could best induce the surface exposure of the peptide on MHC-I, and this is a result disproving that cytotoxic T cells can be most effectively activated in immunotherapy.

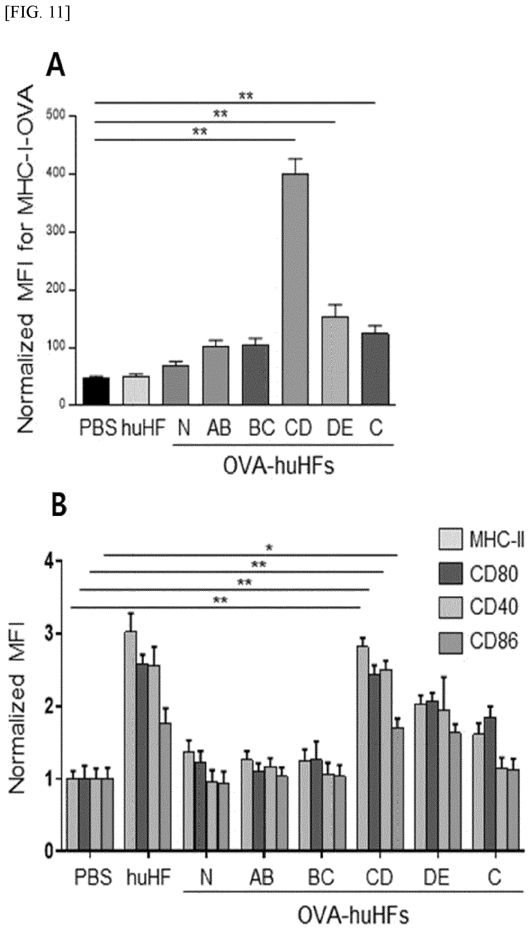

This experiment was carried out through flow cytometry (FACS) (FIG. 11A).

Further, in order to determine whether the huHF protein itself has influence on improvement of the immune response efficiency, the expression rates of MHC-II, CD40, CD80 and CD86 exposed on the surface of the dendritic cells were compared using the same particles.

As a result, it was confirmed that costimulatory effectors were expressed in the order of CD, DE, and C-terminus (FIG. 11B).

## 10. Cancer growth inhibition experiment I (Vaccination; prevention)

Based on the above experimental results, the huHF, huHF-gp100 loops (10 µM) proteins and samples containing only PBS buffer, respectively, were injected into C57BL/6 mice (n=3) three times at an interval of one week by subcutaneous injection. After passing a period of time for the immune response to occur for 1 week, B16F10 cell line was implanted in each mouse and the growth rate of cancer was observed.

A size of cancer cells was calculated by the following Equation:

$$[\text{Equation 4}]$$

$$(\text{Tumor volume}) = (\text{Major axis}) \times (\text{Minor axis})^2 \times 0.52$$

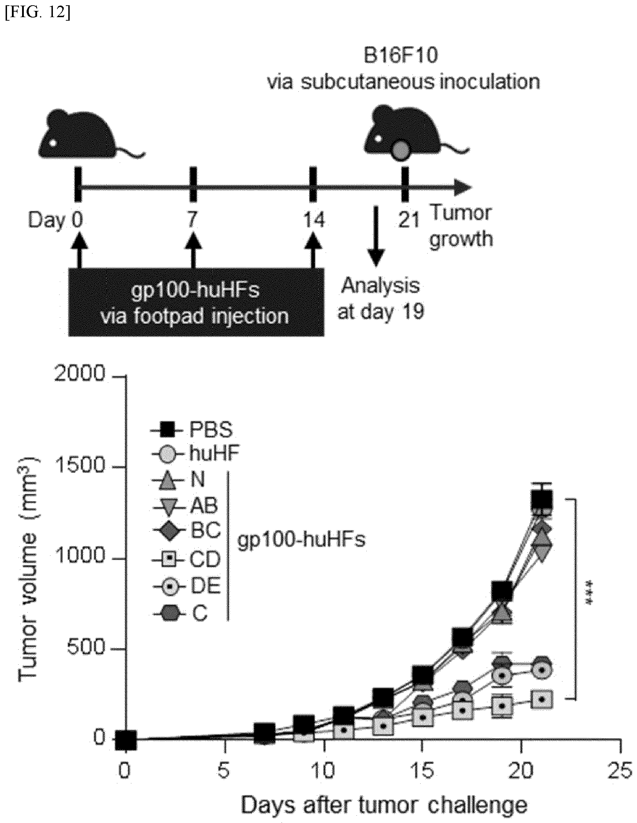

As a result, it was confirmed that effects of inhibiting tumor growth were exhibited in the order of the huHF-CD-gp100, huHF-DE-gp100, and huHF-gp100-C terminal particles (FIG. 12).

## 11. Cancer growth inhibition experiment II (Treatment)

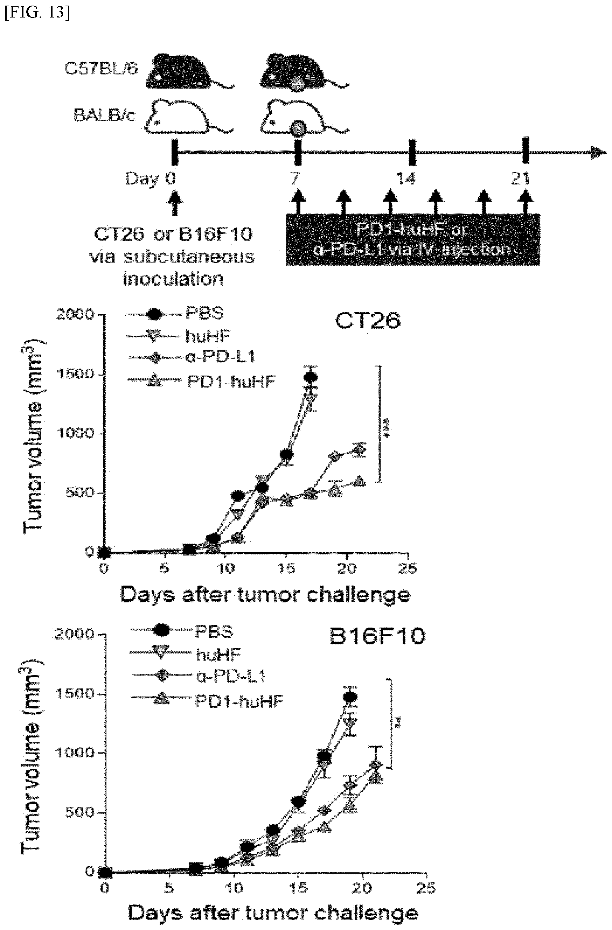

In order to determine whether the huHF-PD1 protein has cancer treatment effects through immune checkpoint suppression compared to the actual antibody therapeutic agents, Balb/c mice having a predetermined size of colon cancer tumors (CT26) were used by the present inventors. Specifically, PBS, PD-L1 antibody, and huHF-PD1 protein were injected intravenously to the mice at an interval of 3 days. As a result of observation, it could be observed that the huHF-PD1 protein showed tumor treatment efficacy similar to the actual antibody therapeutic agents (FIG. 13).

Next, it was investigated whether the first protein (CD loop-huHF) and the second protein (huHF-PD1) have synergistic effects in actual treatment for inhibition of tumor growth *in vivo* and combined treatment. To this end, huHF-CD loop-gp100 and huHF-CD loop-AH1 (10 µM) proteins, in which corresponding cancer-specific antigen epitopes (gp100 and AH1) are inserted into the CD-loop showing the best tumor growth inhibitory effects, were injected to the mice by subcutaneous injection at an interval of 3 days. At the same time, huHF-PD1 (5 µM) and PD-L1 antibody therapeutic agent samples as the control were injected intravenously at an interval of 3 days. The experiment using the huHF-CD loop-gp100 protein has adopted C57BL/6 mice with B16F10 melanoma, while the experiment using the huHF-CD loop-AH1 protein has adopted Balb/c mice with CT26 colon cancer. In each experiment, 5 mice per experimental group were used, and a size of cancer cells was calculated by the following equation:

$$[\text{Equation 4}]$$

$$(\text{Tumor volume}) = (\text{Major axis}) \times (\text{Minor axis})^2 \times 0.52$$

At this time, the experimental groups used herein are: 1) no treatment group; 2) the first protein treatment group (AH1-huHF and gp100-huHF); 3) the antibody therapeutic agent treatment group (α-PD-L1); 4) the second protein treatment group (huHF-PD1); 5) the group administered with a combination of the first protein and the antibody therapeutic agent (AH1-huHF+α-PD-L1 and gp100-huHF+α-PD-L1); and 6) the group administered with a combination of the first protein and the second protein (AH1-huHF+huHF-PD1 and gp100-huHF+huHF-PD1).

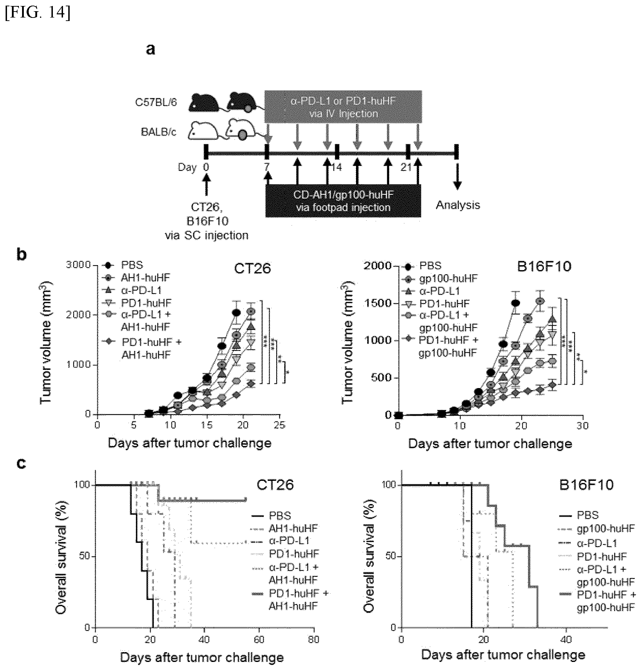

As a result of the experiment, it was confirmed that the experimental group No. 6 treated with the first protein (CD-loop-gp100 or AH1) and the second protein (huHF-PD1) according to the present invention showed the most excellent tumor treatment effects. Further, the survival rate of each experimental group was also measured (FIG. 14).

## 12. *In vitro* immunization experiment to confirm inhibition of cancer growth

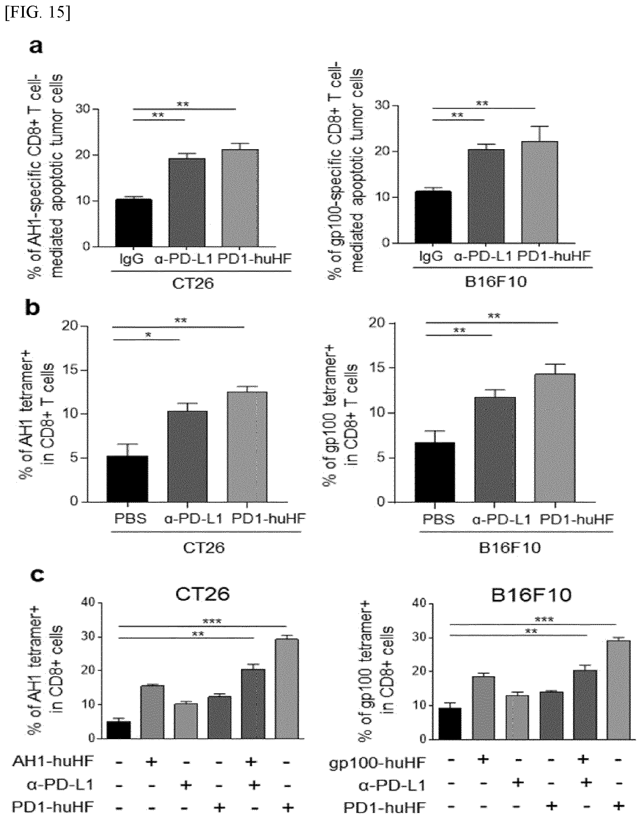

In order to determine whether the huHF-PD1 protein is effective in cancer treatment through immune checkpoint suppression compared to the actual antibody therapeutic agent, PDL1 antibody, the activity response of cells and the cancer cell killing efficiency when the PD-L1 antibody and huHF-PD1 protein react with cancer cells, respectively, were compared by the present inventors. Specifically, after treating colon cancer and melanoma cancer cells with the PDL1 antibody and huHF-PD1 protein, the response of T-cells was observed *in vitro.* From the observation, it was confirmed that IFN-gamma, a specific cytokine capable of killing cancer cells induced by CD8+ cells, was more detected in the experimental group treated with the huH-PD1 protein, as compared to the experimental group treated with the PD-L1 antibody. Moreover, it was additionally confirmed that the cancer cell death rate was also higher. From the above results, it was predicted that the huHF-PD1 protein would be better in terms of cancer cell treatment efficacy than the PD-L1 antibody (FIGS. 15A, B). In addition, the T-cell activity response was observed in the following experimental groups: 1) no treatment group; 2) the first protein treatment group (AH1-huHF and gp100-huHF); 3) the antibody therapeutic agent treatment group (α-PD-L1); 4) the second protein treatment group (huHF-PD1); 5) the group administered with a combination of the first protein and the antibody therapeutic agent (AH1-huHF+α-PD-L1 and gp100-huHF+α-PD-L1); and 6) the group administered with a combination of the first protein and the second protein (AH1-huHF+huHF-PD1 and gp100-huHF+huHF-PD1). As a result, it was also confirmed that T-cell activity is the most excellent in the experimental group No. 6 (AH1-huHF+huHF-PD1 and gp100-huHF+huHF-PD1), which also showed the best result in term of tumor growth inhibition (FIG. 15C).

**13. Comparative experiment on immune side effects of current antibody therapeutic agents and huHF-PD1 as an alternative therapeutic agent developed by the present research team**

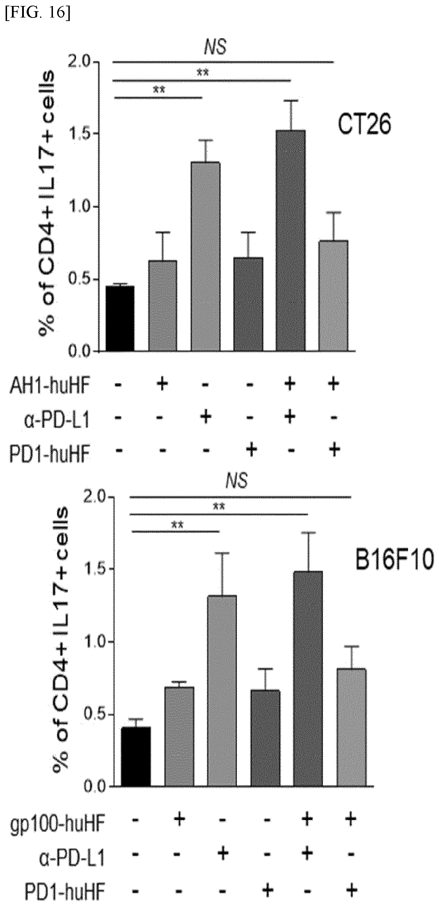

The present inventors have proved that the huHF-PD1 protein has cancer treatment efficacy through immune checkpoint suppression as compared to PDL1 antibody, which is an actual antibody therapeutic agent. At the same time, it was also demonstrated that the degree of induction of immune side effects when injected *in vivo* is also reduced. The most significant problem with the current antibody therapeutic agents is immune side effects caused by long-term accumulation in the body when injecting proteins. In this regard, the most representative cytokine causing the above immune side effects is known as IL-17. Accordingly, the present inventors have implemented an IL-17 detection test using the blood samples of experimental group Nos. 1 to 6 described in Example 11.
As a result, it was confirmed that IL-17 is detected only in the experimental group Nos. 3 and 5 using the antibody therapeutic agent. From the above results, it was confirmed that the protein according to the present invention has lower induction of immune side effects (FIG. 16).

**14. Cancer growth inhibition experiment III (Postoperative rechallenge)**

As a result of the cancer growth inhibition experiment in Example 11, it was confirmed whether the first protein (CD loop-huHF) and the second protein (huHF-PD1) actually suppressed tumor growth *in vivo* and had synergistic effects during combined treatment. Based on the above results, an experiment was implemented to investigate if the cancer recurs even after surgery. At this time, the experimental groups used herein were the same as in Example 11, that is: 1) no treatment group; 2) the first protein treatment group (AH1-huHF); 3) the antibody therapeutic agent treatment group (a-PD-L1); 4) the second protein treatment group (huHF-PD1); 5) the group administered with a combination of the first protein and the antibody therapeutic agent (AH1-huHF+α-PD-L1); and 6) the group administered with a combination of the first protein and the second protein (AH1-huHF+huHF-PD1). Three (3) weeks after it was judged that the tumor grew and the treatment progressed thus to generate tumor-specific immune cells in the body, the tumors of all experimental groups were surgically removed. After that, CT26 colorectal cancer cells were treated again in all experimental groups to observe whether cancer occurred.
As a result, it was confirmed that the no treatment group 1) has cancer continued to grow, while 6) all mice in the group administered with the first protein and the second protein (AH1-huHF+huHF-PD) have no growth of cancer or the cancer disappearing in a few days.
In this experiment, Balb/c mice were used. 5 mice per experimental group were used in each experiment, and a size of cancer cells was calculated by the following Equation:

[Equation 4]

$$\text{(Tumor volume)} = \text{(Major axis)} \times \text{(Minor axis)}^2 \times 0.52$$

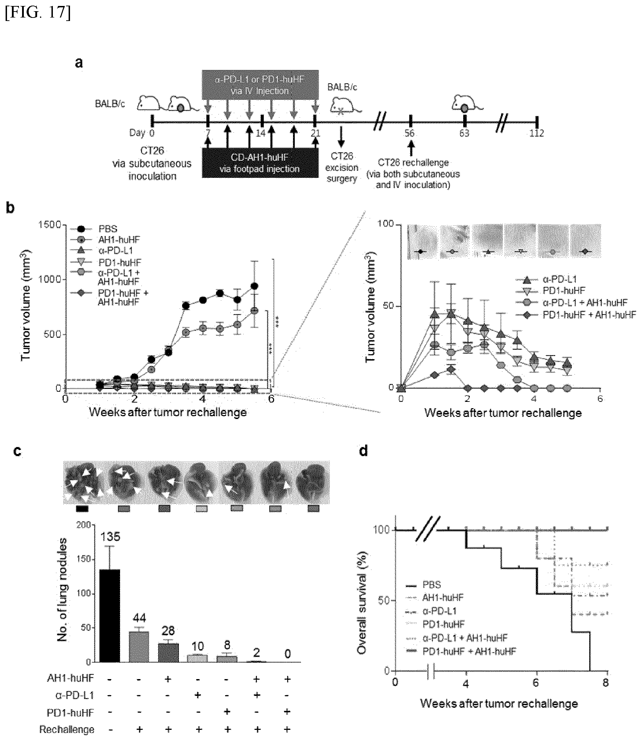

As a result of the experiment, it was confirmed that the experimental group No. 6 which was treated with the first protein (CD-loop-AH1) and the second protein (huHF-PD1) according to the present invention showed the excellent effects of tumor treatment (FIG. 17).

Further, an experiment was conducted to determine whether cancer metastases even after surgery. At this time, the experimental groups used therein were the same as in Example 11, that is: 1) no treatment group; 2) the first protein treatment group (AH1-huHF); 3) the antibody therapeutic agent treatment group (α-PD-L1); 4) the second protein treatment group (huHF-PD1); 5) the group administered with a combination of the first protein and the antibody therapeutic agent (AH1-huHF+α-PD-L1); and 6) the group administered with a combination of the first protein and the second protein (AH1-huHF+huHF-PD1). Three (3) weeks after it was judged that the tumor grew and the treatment progressed thus to generate tumor-specific immune cells in the body, the tumors of all experimental groups were surgically removed. After that, CT26 colorectal cancer cells were treated again in all experimental groups to observe whether cancer occurred.

As a result, it was confirmed that the no treatment group 1) has cancer continued to grow, while 6) all mice in the group administered with the first protein and the second protein (AH1-huHF+huHF-PD) have no growth of cancer or the cancer disappearing in a few days.

In this experiment, Balb/c mice were used. In each experiment, 5 mice were used per experimental group and whether cancer metastases was determined by extracting the lungs of mice in all of the above experimental groups and counting cancer nodules (FIG. 17).

**15. *In vitro* immunization test II to confirm inhibition of cancer growth**

In the same manner as in Example 12, the experimental groups, that is: (1) no treatment group; 2) the first protein treatment group (AH1-huHF and gp100-huHF); 3) the antibody therapeutic agent treatment group (α-PD-L1); 4) the second protein treatment group (huHF-PD1); 5) the group administered with a combination of the first protein and the antibody therapeutic agent (AH1-huHF+α-PD-L1 and gp100-huHF+α-PD-L1); and 6) the group administered with a combination of the first protein and the second protein (AH1-huHF+huHF-PD1 and gp100-huHF+huHF-PD1) were observed to investigate T-cell activity responses.

As a result, even after tumor rechallenge, it was confirmed again that T-cell activity was the most excellent in the experimental group No. 6 (AH1-huHF+huHF-PD1 and gp100-huHF+huHF-PD1), which showed the best result of tumor growth inhibition in Example 12 (FIG. 18).

**16. Preparation of protein to which disease antigen epitopes are fused at various sites of ferritin monomers**

Structures in which gp100 is fused between N-terminus and A helix of ferritin, gp100 is fused between E helix and C-terminus, gp100 is fused inside D helix, and gp100 is fused inside E helix, respectively, were prepared.

The vectors illustrated in FIGS. 19 to 22 and described in Table 2 were prepared according to the method of Example 1. Herein, the primer set of Table 3 was used.

The protein was synthesized according to the method of Example 2, and soluble and insoluble portions were confirmed according to the method of Example 18 described below. Further, it was confirmed that a protein was self-assembled according to the method of Example 4.

**17. Measurement of binding force to transferrin**

The binding force (A) of the prepared protein to transferrin was measured according to the following method.

First, 100 μl of dye that specifically binds to a hexa-His tag (RED-tris-NTA 2nd Generation Dye) was prepared at a concentration of 50 nM, along with 100 μl of the produced protein at a concentration of 200 nM, followed by mixing the same and incubating at room temperature for 30 minutes. The incubated product was centrifuged at 13000 rpm for 10 minutes at 4 °C by a centrifuge, thus to separate the supernatant and obtain a dye-labeled protein.

Then, 25 μl of 9.65 μM transferrin receptor was added to the 1st PCR tube; 10 μl of PBS-T (PBS + 0.5% tween 20) buffer was added to 2nd to 16th PCR tubes; 10 μl of transferrin in the 1st PCR tube was transferred to the 2nd PCR tube; and 10 μl was again transferred from the 2nd PCR tube to the 3rd PCR tube. Likewise, this procedure was continued up to the 16th tube thus to perform 1/2 dilution in a sequential order so that each of the 2nd to 16th PCR tubes becomes 20 μl.

Thereafter, 10 μl of the dye-labeled protein was added to each PCR tube, and the reaction was performed at room temperature for 1 hour.

Subsequently, the reaction solution of each tube was put into the capillary of a microscale thermophoresis device to determine homogeneous fluorescence intensity $F_{cold}$ without laser radiation. Further, the microscale thermophoresis device (Monolith NT.115) was set to provide 40% MST power and LED power such that the acquired fluorescence intensity is within a range of 10,000 to 15,000, while irradiating each capillary with a laser for 30 seconds thus to obtain fluorescence intensity $F_{hot}$ in a heated state.

Thereafter, normalized fluorescence $F_{norm}$ (‰) (= ($F_{hot}/F_{cold}$) × 1000) in each capillary was obtained, from which the capillary in a reaction equilibrium state ("steady state") is found, followed by obtaining a concentration expressed by Equation 1.

[TABLE 5]

| TSA insertion site | TfR | gp100 |
|---|---|---|
| N | Human | 18.484±1.13 |
| N | Mouse | 48.323±2.86 |
| AB | Human | 29.663±0.71 |
| AB | Mouse | 40.787±2.85 |
| BC | Human | 14.043±3.27 |
| BC | Mouse | 48.021±3.37 |
| CD | Human | 4.8943±2.98 |
| CD | Mouse | 15.94±2.52 |
| DE | Human | 9.7809±1.97 |
| DE | Mouse | 30.485±1.11 |
| C | Human | 5.6533±1.33 |
| C | Mouse | 34.795±0.98 |
| EC | Human | 5.6768±1.29 |
| Din | Human | 29.288±3.71 |
| $E_{in}$ | Human | 6.276±1.76 |

[TABLE 6]

| TSA insertion site | TfR | AH1 |
|---|---|---|
| N | Human | 84.648±0.83 |
| AB | Human | 10.933±0.32 |
| BC | Human | 106.64±0.74 |
| CD | Human | 3.4503±3.49 |
| DE | Human | 6.1146±4.11 |
| C | Human | 5.3748±1.25 |

TABLE 7]

| TSA insertion site | TfR | PD1 |
|---|---|---|
| C | Human | 265.84±0.98 |
| C | Mouse | 667.51±2.34 |

[TABLE 8]

| Sample | TfR | Binding force |
|---|---|---|
| Model antigen RFP-huHF(TSA insertion site C) | Human | 127.23±0.71 |
| WT huHF | Human | 2.498±1.77 |
| WT huHF | Mouse | 7.59±2.72 |

**18. Determination of the proportion of water-soluble fraction of protein**

Various expression vectors based on pT7-7 were used for transformation of BL21 (DE3) competent cells. A single colony was inoculated into LB liquid medium (50 mL) added with 100 mg/L of ampicillin, and cultured in a shaking incubator at 37 °C and 130 rpm. When turbidity (turbidity/optical density at 600 nm) reached 0.5, the expression of a target protein was induced through 1 mM IPTG administration. Then, after incubation at 20 °C for 12 to 16 hours, the cells in the culture medium were spun-down through centrifugation (13000 rpm, 10 minutes), and the cell pellets were collected and resuspended in 10 mM Tris-Hcl buffer (pH 7.4). The resuspended cells were crushed using a Branson Sonifier (Branson Ultrasonics Corp., Danbury, CT). After sonication, the supernatant containing a soluble protein and aggregates containing an insoluble protein were separated by centrifugation (13000 rpm, 10 minutes). The separates soluble and insoluble protein fractions were subjected to analysis of solubility through SDS-PAGE. That is, target protein bands stained with Coomassie were scanned with a densitometer (Duoscan T1200, Bio-Rad, Hercules, CA), followed by quantifying a ratio of the water-soluble fraction. Specifically, using the scanned SDS-PAGE gel image, a band thickness and a background value were set by means of 'Quantity One' program and 'Volume Rect. Tool', and then, a sum of the soluble and insoluble protein fractions was set to 100% using the 'Volume Analysis Report', followed by quantification of the solubility.

[TABLE 9]

| Protein | Water-soluble fraction ratio (%) | Protein | Water-soluble fraction ratio (%) |
|---|---|---|---|
| N-OVA-huHF | 89.35 | N-gp100-huHF | 92.48 |
| AB-OVA-huHF | 93.81 | AB-gp100-huHF | 93.86 |
| BC-OVA-huHF | 95.74 | BC-gp100-huHF | 94.43 |
| CD-OVA-huHF | 98.73 | CD-gp100-huHF | 95.57 |
| DE-OVA-huHF | 96.82 | DE-gp100-huHF | 95.39 |
| C-OVA-huHF | 96.62 | C-gp100-huHF | 96.40 |
| N-AH1-huHF | 81.74 | NA-gp100-huHF | 67.22 |
| AB-AH1-huHF | 94.63 | EC-gp100-huHF | 96.27 |
| BC-AH1-huHF | 92.53 | $D_{in}$-gp100-huHF | 48.21 |
| CD-AH1-huHF | 98.47 | $E_{in}$-gp100-huHF | 93.00 |
| DE-AH1-huHF | 98.71 | PD1-huHF | 74.25 |
| C-AH1-huHF | 87.90 | RFP-huHF | 98.31 |

**19. Use of molecules that bind to immune checkpoint molecules**

(1) After producing a protein in which mouse small PD1 (SEQ ID NO: 8) was fused at the C-terminus of huHF, the efficacy of the protein was investigated (FIG. 23).
The protein was synthesized according to the method of Example 2, and the soluble and insoluble portions were confirmed according to the method of Example 19. Further, it was confirmed that the protein is self-assembled according to the method of Example 4.
The binding force of the produced protein to the transferrin receptor was measured according to the method of Example 17, and a concentration represented by Equation 1 was found to be 44.649±1.34 nM.
The tumor inhibitory ability of the protein was evaluated according to the method of Example 11.
Specifically, PBS, PD-L1 antibody, huHF-PD1, and huHF-msmPD1 proteins, respectively, were injected intravenously to Balb/c mice having a predetermined size of colon cancer tumors (CT26) at an interval of 3 days. As a result of observation, it could be seen that the huHF-msmPD1 protein showed tumor treatment efficacy similar to the antibody therapeutic agents. The experiment has used 3 mice per experimental group, and a size of cancer cells was calculated by the following Equation:

[Equation 4]

$$(\text{Tumor volume}) = (\text{Major axis}) \times (\text{Minor axis})^2 \times 0.52$$

At this time, the experimental groups used herein are: 1) a PBS group, 2) an antibody therapeutic agent treatment group ($\alpha$-PD-L1), 3) a first protein treatment group (huHF-PD1), and 4) a second protein treatment group (huHF-msmPD1).

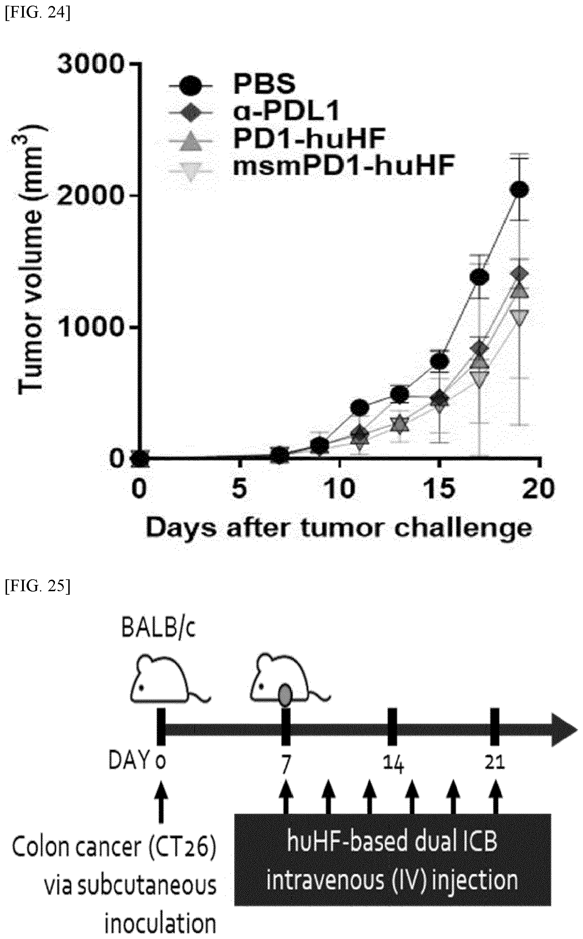

The results are shown in FIG. 24.

Referring to FIG. 24, it is possible to confirm excellent anticancer activity when using the ferritin in which the immune checkpoint molecule-binding molecule is fused.

(2) After producing a protein in which hsmPD1 was fused at the C-terminus of huHF, the efficacy of the protein was investigated.

huHF is a substitution of some amino acids at the binding site (existing in the BC loop) with transferrin, and the protein in which amino acids 81 and 83 in the sequence of SEQ ID NO: 1 are substituted with alanine was used.

This was obtained by mixing the forward primer (SEQ ID NO: 39), 10 $\mu$M of the reverse primer (SEQ ID NO: 40), and huHF-hsmPD1 as a template DNA in Q5 Hot Start High-Fidelity 2X Master Mix, followed by gene mutation. Then, a protein was obtained according to the method of Example 2.

As hsmPD1, the sequence of SEQ ID NO: 41 was used.

The binding force of the produced protein to h-PD-L1 and m-PD-L1 was measured according to the method of Example 17. As a result, it was found that the binding force to h-PD-L1 is 13.417$\pm$1.97 nM, and the binding force to m-PD-L1 is 177.14$\pm$3.32 nM.

(3) After producing a protein in which molecules binding to immune checkpoint molecules PD-L1 and TIGIT were fused to ferritin, and the efficacy of the protein was investigated.

As the immune checkpoint molecule-binding molecule, HCDR3 sequence of the antibody was used, and the sequence used herein is shown in Table 10 below.

[TABLE 10]

| Sequence No. | Designation |
|---|---|
| 42 | huHF-$\alpha$PD-L1* |
| 43 | huHF-$\alpha$TIGIT* |

[TABLE 11]

| Protein | Expression vector |
|---|---|
| huHF-PD-L1-TIGIT dual blocker | BC(92D/93W): NH2-NdeI-(His)6-huHF-[$\alpha$TIGIT HCDR3]-huHF-$\alpha$PD-L1 HCDR3-HindIII-COOH |

[TABLE 12]

| Sequence No. | Designation | Insertion site |
|---|---|---|
| 44 | BC_$\alpha$_PD-L1_F | BC loop |
| 45 | BC_$\alpha$_PD-L1_R | |
| 46 | C_$\alpha$_TIGIT F | C-terminus |
| 47 | C_$\alpha$_TIGIT R | |

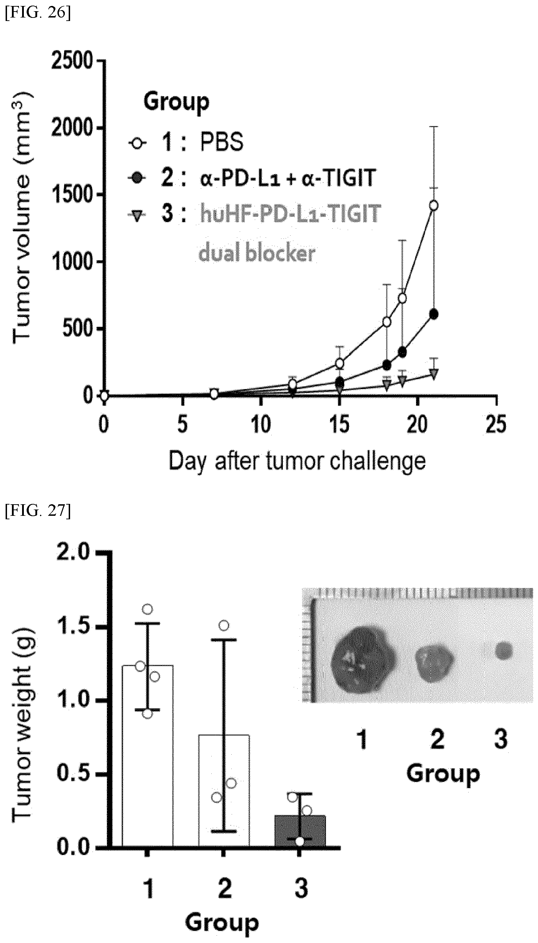

The vector of Table 8 was prepared according to the method of Example 1, and the primer set of Table 9 was used. The protein was synthesized according to the method of Example 2. The tumor suppressing ability of the protein was determined by subcutaneous inoculation of a colon cancer cell line (CT26) into BALB/c mice and injecting the protein according to the schedule of FIG. 25, followed by evaluation according to the method of Example 11 (FIG. 26).

Specifically, PBS, PD-L1 antibody, TIGIT antibody, and huHF-PD-LI-TIGIT dual blocker proteins were injected intravenously to Balb/c mice having a predetermined size of colon cancer tumors (CT26) at an interval of 3 days. As a result of observation, it could be seen that the huHF-PD-LI-TIGIT dual blocker protein showed tumor treatment efficacy similar to the antibody therapeutic agents. 4 mice per experimental group were used in each experiment, and a size of cancer cells was calculated by the following equation:

[Equation 4]

$$(\text{Tumor volume}) = (\text{Major axis}) \times (\text{Minor axis})^2 \times 0.52$$

At this time, the experimental groups used herein are: 1) a PBS group, 2) an antibody therapeutic agent combined treatment group ($\alpha$-PD-L1, $\alpha$-TIGIT), and 3) a protein treatment group (huHF-PD-L1-TIGIT dual blocker).

Further, tumor tissues were removed for each treatment group and the weight thereof was measured, and the results are shown in FIG. 27. From the results, it is possible to confirm the excellent anticancer efficacy of the protein in which the molecules binding to PD-L1 and TIGIT are fused.

(4) Analysis of the efficiency according to the fusion site of the immune checkpoint molecule-binding molecule to the ferritin monomer

A protein in which $\alpha$-PD-L1 HCDR3 is fused at different sites of a ferritin monomer was produced, followed by investigating the tumor suppression ability.

The protein in which $\alpha$-PD-L1 HCDR3 is fused at the AB loop, BC loop, CD loop, DE loop, and C-terminus was produced (AB loop among huHF 5T to 176G; between 45D/46V, BC loop; 92D/93W, CD loop; 126D/127P, DE loop; 162E/163S, based on PDB 3AJO sequence). This was produced in the same manner as in Examples 1 and 2, except that the sequence of Table 10 above was used.

The ability of the prepared protein to target colorectal cancer cells was confirmed by the method of Example 6.

Specifically, in order to compare the efficiency of FITC fluorescent substance-adhered huHF-$\alpha$PD-L1 HCDR3 (AB, BC, CD, DE loops, C-terminus) proteins to target CT26 colorectal cancer, respectively, CT26 colorectal cancer cells were reacted with each of the proteins at a concentration of 300 nM, followed by comparing the fluorescence signals to confirm the cell uptake efficiency. It was confirmed that the huHF-$\alpha$PD-L1 HCDR3 proteins (AB, BC, CD, DE loops, C-terminus) were bonded to the cancer cells and showed fluorescent signals rather than the control huHF protein.

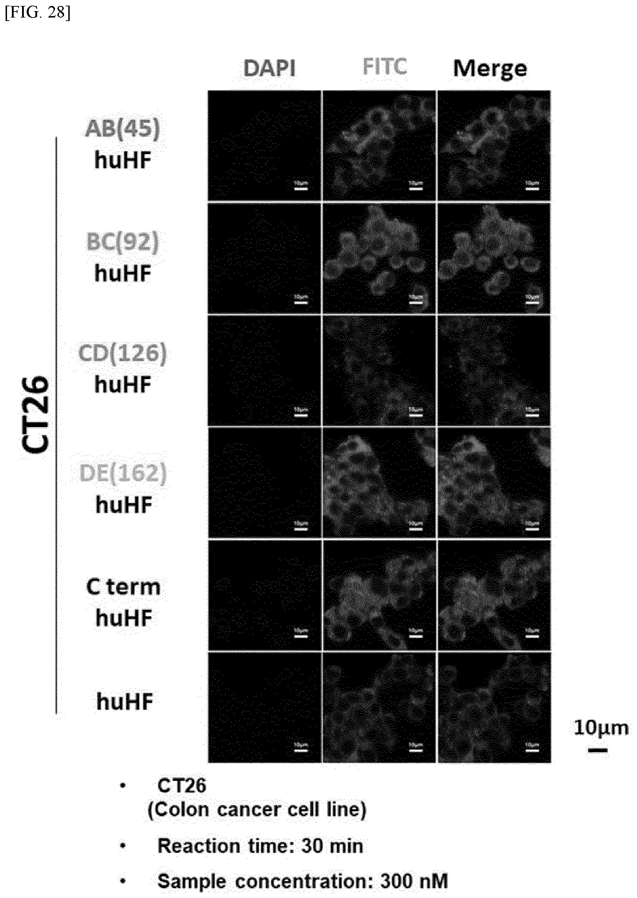

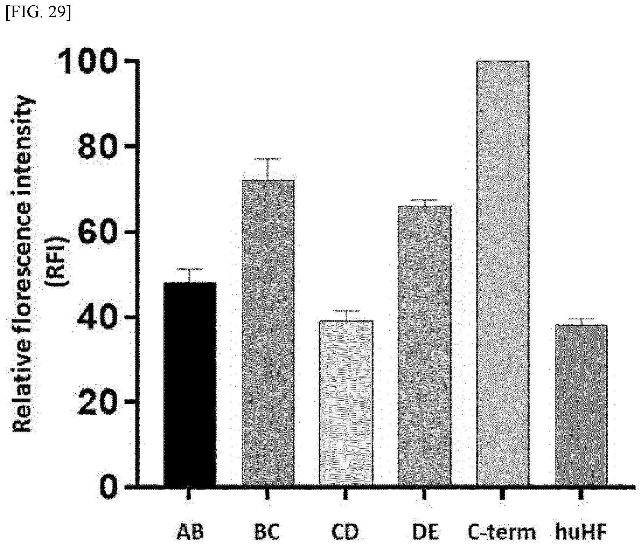

The results are shown in FIG. 28, and the relative fluorescence intensity thereof is shown in FIG. 29.

As a result, it was confirmed that, regardless of the fusion site, a stronger targeting ability was exhibited than the huHF protein.

**20. Use of antibody CDR as an immune checkpoint molecule-binding molecule**

**(1) Construction of expression vector for protein production**

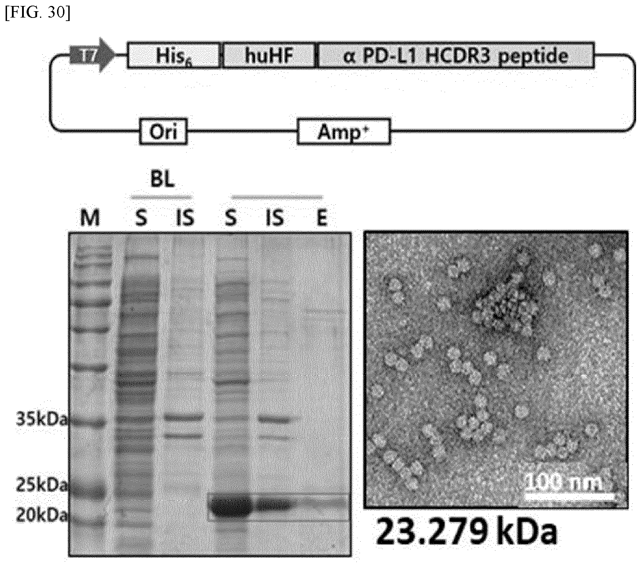

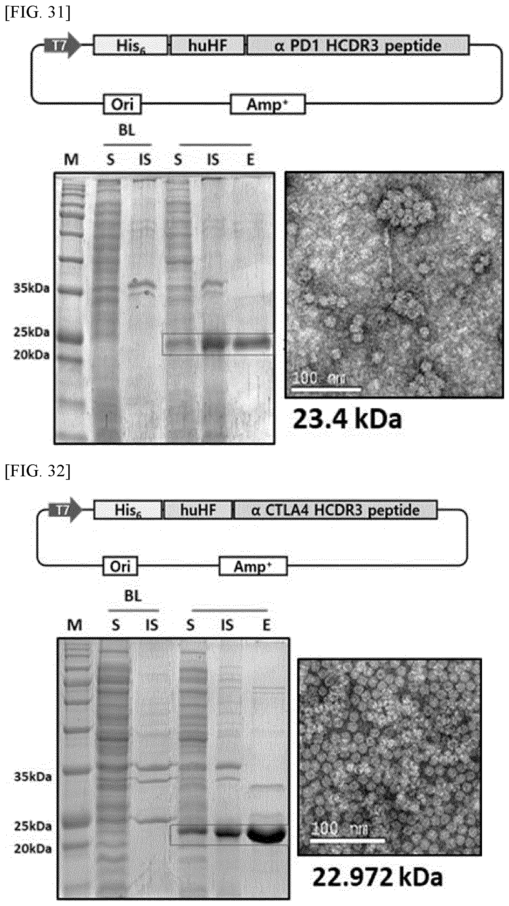

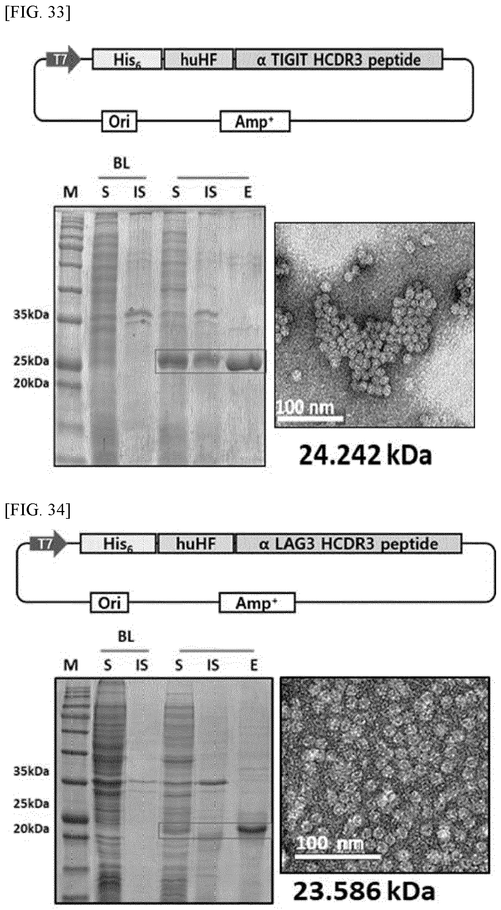

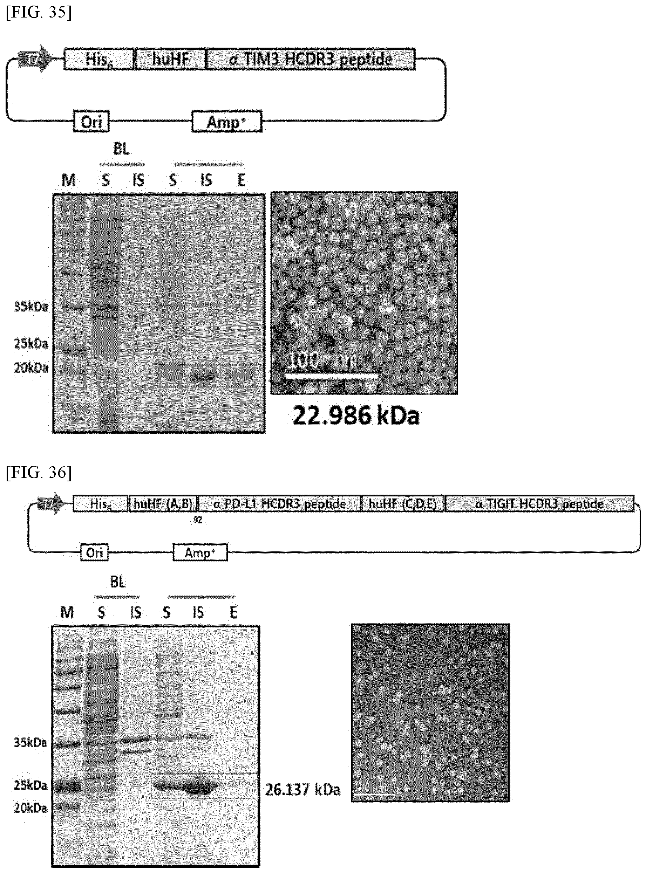

The sequence of Table 13 below was used, PCR was implemented according to the vector schematic diagrams of FIGS. 29 to 36 and Table 14 below, and huHF-$\alpha$PD1 HCDR3 (C-terminus), huHF-$\alpha$CTLA4 HCDR3 (C-terminus), huHF $\alpha$TIGIT HCDR3 (C-terminus), huHF-$\alpha$LAG3 HCDR3 (C-terminus), huHF-$\alpha$TIM3 HCDR3 (C-terminus), huHF-$\alpha$PD-L1 HCDR3 (AB loop)-aTIGIT HCDR3 (C-terminus) (dual blocker) were prepared. All the prepared plasmid expression vectors were purified on an agarose gel, and then the sequence was confirmed through complete DNA sequencing.

Specifically, using the primer sets in Table 15, PCR products required for preparation of each expression vector were sequentially inserted into the plasmid pT7-7 vector to construct an expression vector capable of expressing nanoparticles of each protein.

[TABLE 13]

| Sequence No. | Designation |
|---|---|
| 48 | huHF-$\alpha$PD-L1 |
| 49 | huHF-$\alpha$PD1 |
| 50 | huHF-$\alpha$CTLA4 |
| 51 | huHF-$\alpha$LAG3 |
| 52 | huHF-$\alpha$TIM3 |
| 53 | huHF $\alpha$TIGIT |

EP 4 053 157 A1

[TABLE 14]

| Protein | Expression vector |
|---|---|
| huHF-αPD-L1 | NH2-NdeI-huHF-αPD-L1 HCDR3-HindIII-COOH |
| huHF-αPD1 | NH2-NdeI-huHF-αPD1 HCDR3 -HindIII-COOH |
| huHF -αCTLA4 | NH2-NdeI-huHF-αCTLA4 HCDR3-HindIII-COOH |
| huHF -αLAG3 | NH2-NdeI-huHF-αLAG3 HCDR3-HindIII-COOH |
| huHF -αTIM3 | NH2-NdeI-huHF-αTIM3 HCDR3 -HindIII-COOH |
| huHF-aTIGIT | NH2-NdeI-huHF-αTIGIT HCDR3-HindIII-COOH |
| huHF-PD-L1-TIGIT dual blocker | BC(92D/93W): NH2-NdeI-(His)6-huHF-[aTIGIT HCDR3]-huHF- αPD-L1 HCDR3-HindIII-COOH |

[TABLE 15]

| Sequence No. | Designation | Insertion site |
|---|---|---|
| 54 | α_PD-L1_F | C-terminus |
| 55 | α_PD-L1_R | |
| 56 | α_PD1_F | C-terminus |
| 57 | α_PD1_R | |
| 58 | α_CTLA4_F | C-terminus |
| 59 | α_CTLA4_R | |
| 60 | α_LAG3_F | C-terminus |
| 61 | α_LAG3-R | |
| 62 | α_TIM3_F | C-terminus |
| 63 | α_TIM3 R | |
| 64 | α_TIGIT_F | C-terminus |
| 65 | α_TIGIT_R | |
| 66 | BC_α_PD-L1_F | BC loop |
| 67 | BC_α_PD-L1_R | |

**(2) Verification of protein synthesis, purification and assembly**

Proteins were produced and water-soluble fractions were confirmed in the same manner as in Examples 2 to 4. Further, it was confirmed in the same manner as in Example 5 whether or not spherical nanoparticles were formed (FIGS. 29 to 36).

**(3) Measurement of binding force to antigen**

The binding force to an antigen was measured in the same manner as in Example 6, except that the antigen for each antibody was used.

The binding force of the antibody is shown in Table 16, and the binding force of each of the proteins in the example is shown in Tables 17 and 18. Referring to these tables, it can be seen that the proteins of the examples exhibit excellent binding ability with human antigens.

24

[TABLE 16]

| Human Ab | Kd (nM) | Catalog # |
|---|---|---|
| αPD-L1 | 5.8227±0.52 | 10084-MM02(Sino biological) |
| αPD1 | 9.2096±1 | MBS154625 (Mybiosource) |
| αCTLA4 | 3.4228±1.81 | 11159-MM06(Sino biological) |
| αTIM3 | 7.9993±1.01 | MBS4156568(Mybiosource) |
| αTIGIT | 10.32±1.27 | MBS 154627(Mybiosource) |

[TABLE 17]

| Sample | murine IC molecule(standard) | Kd(nM) |
|---|---|---|
| huHF-αPD-L1 | PD-L1 | 3.1692±2.56 |
| huHF-αPD1 | PD1 | 87.889±2.47 |
| huHF -αCTLA4 | CTLA4 | 17.513±0.462 |
| huHF -αLAG3 | LAG3 | 37.817±1.82 |
| huHF -αTIM3 | TIM3 | 7.0831±1.64 |
| huHF-aTIGIT | TIGIT | 3.9997±2.29 |
| huHF-α PD-L1-αTIGIT | PD-L1 | 4.9956±2.79 |
| | TIGIT | 4.7343±2.52 |

[TABLE 18]

| Sample | human IC molecule(standard) | Kd(nM) |
|---|---|---|
| huHF-αPD-L1 | PD-L1 | 10.708±4.52 |
| huHF-αPD1 | PD1 | 17.776±4.38 |
| huHF-αCTLA4 | CTLA4 | 10.167±3.11 |
| huHF-αLAG3 | LAG3 | 18.498±3.17 |
| huHF-αTIM3 | TIM3 | 13.03±4.21 |
| huHF-aTIGIT | TIGIT | 7.1276±2.16 |
| huHF-αPD-L1- | PD-L1 | 3.5605±1.07 |
| αTIGIT | TIGIT | 6.6423±3.46 |

## Claims

1. A protein formed by self-assembly of ferritin monomers to which disease antigen epitopes are fused, wherein a binding force (K) to a human transferrin receptor satisfies the following Equation 1:

$$[\text{Equation } 1]$$

$$K \leq 125 \text{ nM}$$

(wherein K = [P][T]/[PT], wherein [P] represents a concentration of the protein in an equilibrium state of a binding reaction between the protein and the human transferrin receptor, [T] represents a concentration of the human transferrin receptor in the equilibrium state, and [PT] represents a concentration of a complex of the protein and the human transferrin receptor in the equilibrium state).

2. The protein according to claim 1, wherein the protein has $K \leq 100$ nM.

3. The protein according to claim 1, wherein the protein has $K \leq 50$ nM.

4. The protein according to claim 1, wherein the disease antigen epitope is any one selected from the group consisting of gp100, MART-1, Melna-A, MAGE-A3, MAGE-C2, Mammaglobin-A, proteinsase-3, mucin-1, HPV E6, LMP2, PSMA, GD2, hTERT, PAP, ERG, NA17, ALK, GM3, EPhA2, NA17-A, TRP-1, TRP-2, NY-ESO-1, CEA, CA 125, AFP, Survivin, AH1, ras, G17DT, MUC1, Her-2/neu, E75, p53, PSA, HCG, PRAME, WT1, URLC10, VEGFR1, VEGFR2, E7, Tyrosinase peptide, B16F10, EL4 and neoantigens.

5. The protein according to claim 1, wherein the ferritin is a human ferritin heavy chain.

6. The protein according to claim 1, wherein the protein has a spherical shape in which 24 ferritin monomers are self-assembled.

7. The protein according to claim 1, wherein the disease antigen epitope is fused to at least one of sites between adjacent α-helixes of the ferritin monomer.

8. The protein according to claim 1, wherein the disease antigen epitope is fused at N-terminus or C-terminus of the ferritin monomer.

9. The protein according to claim 1, wherein the disease antigen epitope is fused to A-B loop, B-C loop, C-D loop or D-E loop of the ferritin monomer.

10. The protein according to claim 1, wherein the disease antigen epitope is fused between N-terminus and A helix or between E helix and C-terminus of the ferritin monomer.

11. The protein according to claim 1, wherein the disease antigen epitope is fused inside at least one of the helixes of the ferritin monomer.

12. The protein according to claim 1, wherein the disease antigen epitope has an amino acid length of 25aa or less.

13. The protein according to claim 1, wherein the protein contains a water-soluble fraction which is present in a ratio of 40% or more in an *E. coli* production system.

14. The protein according to claim 1, wherein the disease antigen epitope is any one selected from the group consisting of brain cancer, head and neck cancer, bladder cancer, breast cancer, cervical cancer, colon cancer, colorectal cancer, endometrial cancer, esophageal cancer, leukemia, lung cancer, liver cancer, ovarian cancer, pancreatic cancer, prostate cancer, rectal cancer, kidney cancer, stomach cancer, testicular cancer, uterine cancer, vascular tumor, squamous cell carcinoma, adenocarcinoma, small cell carcinoma, melanoma, glioma, neuroblastoma, sarcoma, laryngeal cancer, parotid carcinoma, biliary tract cancer, thyroid cancer, actinic keratosis, acute lymphocytic leukemia, acute myeloid leukemia, adenocarcinoma, adenoma, glandular squamous cell carcinoma, anal duct cancer, anal cancer, anal rectal cancer, astrocytoma, large vaginal gland carcinoma, basal cell carcinoma, biliary cancer, bone cancer, bone marrow cancer, bronchial cancer, bronchial gland carcinoma, carcinoid, bile duct carcinoma, chronic lymphocytic leukemia, chronic myelogenous leukemia, clear cell carcinoma, connective tissue cancer, cyst adenoma, digestive system cancer, duodenal cancer, endocrine system cancer, endoderm sinus tumor, endometrial hyperplasia, endometrial adenocarcinoma, endothelial cell carcinoma, ventricular cell, epithelial cell cancer, orbital cancer, focal nodular hyperproliferation, gallbladder cancer, flank cancer, gastric basal cancer, gastrinoma, glioblastoma, glucagonoma, heart cancer, hemangioblastoma, hemangioendothelioma, hemangioma, hepatoadenoma, hepatic adenoma, hepatobiliary cancer, hepatocellular carcinoma, Hodgkin's disease, ileal cancer, insulinoma, intraepithelial neoplasm, intraepithelial squamous cell neoplasm, intrahepatic biliary cancer, invasive squamous cell carcinoma, jejunal cancer, joint cancer, pelvic cancer, giant cell carcinoma, colon cancer, lymphoma, malignant mesothelioma, mesothelioma, medullary epithelial carcinoma, meningeal cancer, mesothelial cancer, metastatic carcinoma, oral cancer, mucosal epithelial carcinoma, multiple myeloma, muscle cancer, nasal duct cancer, nervous system cancer, non-epithelial skin cancer, non-Hodgkin's lymphoma, chondrocyte carcinoma, oligodendrocyte cancer, oral cancer, osteosarcoma, papillary serous adenocarcinoma, penile cancer, pharyngeal cancer, pituitary tumor, plasmacytoma, sarcoma, pulmonary blastoma, rectal cancer, renal cell carcinoma, respiratory cancer, retinoblastoma, serous carcinoma, sinus cancer, skin cancer, small cell carcinoma, small intestine cancer, smooth muscle cancer, soft tissue cancer, somatostatin-secreting tumor, spinal cancer, squamous cell carcinoma, striatal muscle cancer, subcutaneous cell carcinoma, T cell leukemia, tongue cancer, ureteral cancer, urethral cancer, cervical cancer, uterine trunk cancer, vaginal cancer, VIPoma, genital cancer, hyperdifferentiated carcinoma and Wilm's tumor.

15. A pharmaceutical composition for prevention or treatment of cancer, comprising the protein according to any one of claims 1 to 14.

16. The pharmaceutical composition according to claim 15, wherein the cancer is any one selected from the group consisting of melanoma, lung cancer, colon cancer, liver cancer, glioblastoma, ovarian cancer, colon cancer, head and neck cancer, bladder cancer, renal cell cancer, stomach cancer, breast cancer, metastatic cancer, prostate cancer, gallbladder cancer, pancreatic cancer and blood cancer.

17. The pharmaceutical composition according to claim 15, wherein the pharmaceutical composition is an injectable formulation.

18. The pharmaceutical composition according to claim 15, wherein the pharmaceutical composition is administered through intraperitoneal, intravenous, intramuscular, subcutaneous, intradermal, oral, topical, intranasal, pulmonary or rectal administration.

[FIG. 1]

**A** vector construction

**B** Structure

[FIG. 2]

[FIG. 3]

[FIG. 4]

| Protein NP | Kd (nM) |
|---|---|
| huHF | 7.59 ± 2.7 |
| N | 48.32 ± 2.87 |
| AB | 40.79 ± 2.85 |
| BC | 48.02 ± 3.37 |
| CD | 15.94 ± 2.51 |
| DE | 30.48 ± 1.11 |
| C | 34.79 ± 0.97 |

[FIG. 5]

[FIG. 6]

|  | DAPI | Cy5.5 | Merge |
|---|---|---|---|
| huHF | | | |
| N-gp100 -huHF | | | |
| AB-gp100 -huHF | | | |

|  | DAPI | Cy5.5 | Merge |
|---|---|---|---|
| BC-gp100 -huHF | | | |
| CD-gp100 -huHF | | | |
| DE-gp100 -huHF | | | |
| C-gp100 -huHF | | | |

10 µm

[FIG. 7a]

[FIG. 7b]

[FIG. 7c]

[FIG. 8]

[FIG. 9]

EP 4 053 157 A1

[FIG. 10]

[FIG. 11]

[FIG. 12]

[FIG. 13]

[FIG. 14]

[FIG. 15]

[FIG. 16]

[FIG. 17]

[FIG. 18]

[FIG. 19]

## - NA-gp100-huHF

[FIG. 20]

# - EC-gp100-huHF

EP 4 053 157 A1

[FIG. 21]

- D<sub>in</sub>-gp100-huHF

[FIG. 22]

- E$_{in}$-gp100-huHF

[FIG. 23]

28.279 kDa

[FIG. 24]

[FIG. 25]

[FIG. 26]

[FIG. 27]

[FIG. 28]

- CT26
  (Colon cancer cell line)
- Reaction time: 30 min
- Sample concentration: 300 nM

[FIG. 29]

[FIG. 30]

23.279 kDa

[FIG. 31]

23.4 kDa

[FIG. 32]

22.972 kDa

[FIG. 33]

**24.242 kDa**

[FIG. 34]

**23.586 kDa**

EP 4 053 157 A1

[FIG. 35]

22.986 kDa

[FIG. 36]

26.137 kDa

## INTERNATIONAL SEARCH REPORT

| International application No. |
| --- |
| **PCT/KR2020/015164** |

**A.    CLASSIFICATION OF SUBJECT MATTER**

**C07K 14/47**(2006.01)i; **C07K 16/28**(2006.01)i; **A61K 39/00**(2006.01)i; **A61K 39/385**(2006.01)i; **A61K 9/51**(2006.01)i; **A61K 9/19**(2006.01)i; **A61K 9/00**(2006.01)i; **A61P 35/00**(2006.01)i; **B82Y 5/00**(2011.01)n

According to International Patent Classification (IPC) or to both national classification and IPC

**B.    FIELDS SEARCHED**

Minimum documentation searched (classification system followed by classification symbols)

C07K 14/47(2006.01); A61K 31/69(2006.01); A61K 47/69(2017.01); A61K 9/51(2006.01); C07K 19/00(2006.01); C12N 15/62(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 페리틴(ferritin), 에피토프(epitope), 종양 항원(tumor antigen), 트랜스페린 수용체(transferrin receptor), 수지상 세포(dendritic cell), 자가조립(self-assembly), 면역관문(immune checkpoint)

**C.    DOCUMENTS CONSIDERED TO BE RELEVANT**

| Category* | Citation of document, with indication, where appropriate, of the relevant passages | Relevant to claim No. |
| --- | --- | --- |
| X | KR 10-2015-0088597 A (UNIST ACADEMY-INDUSTRY RESEARCH CORPORATION) 03 August 2015 (2015-08-03)<br>       See claims 1, 9 and 20; paragraphs [0002]-[0097]; figure 7; and SEQ ID NOs. 4 and 5. | 1-18 |
| X | KR 10-2017-0047120 A (KOREA UNIVERSITY RESEARCH AND BUSINESS FOUNDATION et al.) 04 May 2017 (2017-05-04)<br>       See claims 1-21; paragraphs [0069]-[0106]; and SEQ ID NOs. 1 and 2. | 1-18 |
| A | KR 10-2018-0008353 A (KOREA INSTITUTE OF SCIENCE AND TECHNOLOGY) 24 January 2018 (2018-01-24)<br>       See claims 1-28; paragraphs [0014]-[0141]; and figure 1. | 1-18 |
| A | KR 10-2019-0115262 A (THE INDUSTRY & ACADEMIC COOPERATION IN CHUNGNAM NATIONAL UNIVERSITY (IAC)) 11 October 2019 (2019-10-11)<br>       See claims 1-11. | 1-18 |

☑ Further documents are listed in the continuation of Box C.       ☑ See patent family annex.

| * Special categories of cited documents: | "T" later document published after the international filing date or priority date and not in conflict with the application but cited to understand the principle or theory underlying the invention |
| --- | --- |
| "A"   document defining the general state of the art which is not considered to be of particular relevance | |
| "D"   document cited by the applicant in the international application | "X"   document of particular relevance; the claimed invention cannot be considered novel or cannot be considered to involve an inventive step when the document is taken alone |
| "E"   earlier application or patent but published on or after the international filing date | |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| **17 February 2021** | **17 February 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

International application No.

**PCT/KR2020/015164**

### C. DOCUMENTS CONSIDERED TO BE RELEVANT

| Category* | Citation of document, with indication, where appropriate, of the relevant passages | Relevant to claim No. |
|---|---|---|
| A | LEE, Bo-Ram et al. Engineered human ferritin nanoparticles for direct delivery of tumor antigens to lymph node and cancer immunotherapy. Scientific Reports. 11 October 2016, vol. 6, Article no. 35182, inner pp. 1-12.<br>    See inner pages 1-12. | 1-18 |

Form PCT/ISA/210 (second sheet) (July 2019)

## INTERNATIONAL SEARCH REPORT

| International application No. |
|---|
| **PCT/KR2020/015164** |

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

**INTERNATIONAL SEARCH REPORT**
**Information on patent family members**

| International application No. |
| --- |
| **PCT/KR2020/015164** |

| Patent document cited in search report | | | Publication date (day/month/year) | Patent family member(s) | | | Publication date (day/month/year) |
| --- | --- | --- | --- | --- | --- | --- | --- |
| KR | 10-2015-0088597 | A | 03 August 2015 | KR | 10-1630858 | B1 | 15 June 2016 |
| KR | 10-2017-0047120 | A | 04 May 2017 | US | 10206987 | B2 | 19 February 2019 |
| | | | | US | 2017-0112910 | A1 | 27 April 2017 |
| KR | 10-2018-0008353 | A | 24 January 2018 | CA | 3040440 | A1 | 18 January 2018 |
| | | | | CN | 109803642 | A | 24 May 2019 |
| | | | | EP | 3501509 | A1 | 26 June 2019 |
| | | | | KR | 10-1953617 | B1 | 23 May 2019 |
| | | | | US | 2019-0216947 | A1 | 18 July 2019 |
| | | | | WO | 2018-012952 | A1 | 18 January 2018 |
| KR | 10-2019-0115262 | A | 11 October 2019 | KR | 10-2112759 | B1 | 19 May 2020 |
| | | | | WO | 2019-194393 | A1 | 10 October 2019 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10206987 B